# EUROPÄISCHE PATENTSCHRIFT

(11) **EP 3 851 149 B1**
(45) Veröffentlichungstag und Bekanntmachung des Hinweises auf die Patenterteilung: **05.10.2022**
(21) Anmeldenummer: 21150133.3
(22) Anmeldetag: 05.01.2021
(51) Int. Cl.: A61M 16/08, A61M 16/10, A61M 16/18, A61M 16/20, A61M 16/00, G01F 1/00, G01N 33/00

(54) **ANÄSTHESIEMITTELDOSIERVORRICHTUNG UND VERFAHREN ZUM EINSTELLEN EINER ANÄSTHESIEMITTELKONZENTRATION**
ANESTHETIC AGENT METERING DEVICE AND METHOD FOR ADJUSTING ANESTHETIC AGENT CONCENTRATION
APPAREIL DE DOSAGE D'AGENT ANESTHÉSIQUE ET PROCÉDÉ DE RÉGLAGE D'UNE CONCENTRATION D'AGENT ANESTHÉSIQUE

(30) Priorität: 17.01.2020 DE 102020000267
(43) Veröffentlichungstag der Anmeldung: 21.07.2021
(73) Patentinhaber: Drägerwerk AG & Co. KGaA, 23558 Lübeck (DE)
(72) Erfinder: Bartels, Uwe, 23558 Lübeck (DE); Wruck, Norbert, 23558 Lübeck (DE)

(56) Entgegenhaltungen:
- EP-A2- 0 722 748
- WO-A1-2009/062540
- DE-A1- 4 105 971
- US-A- 4 770 168

## Beschreibung

Die Erfindung betrifft eine Anästhesiemitteldosiervorrichtung zum Einstellen einer Anästhesiemittelkonzentration in einem einem Anästhesieatemkreis bereitzustellenden Gasgemisch. Weiter betrifft die Erfindung ein Verfahren zum Einstellen einer Anästhesiemittelkonzentration in einem einem Anästhesieatemkreis bereitzustellenden Gasgemisch.

Die Verwendung einer Anästhesiemitteldosiervorrichtung zum Anreichern eines bereitgestellten Atemgases mit einem Anästhesiemittel und zum Ausgeben eines derart angereicherten Gasgemisches an einen mit einem Patienten verbundenen Anästhesieatemkreis ist bekannt. Hierbei wird typischerweise ein erster Gaszweig mit einem Anästhesiemittelverdunster verwendet, um das zugeführte Atemgas mit dem Anästhesiemittel anzureichern. Weiterhin wird typischerweise ein zweiter Gaszweig, auch Bypass-Zweig genannt, an dem Anästhesiemittelverdunster vorbeigeführt, um im Rahmen einer Zuführung beider Gaszweige zu einer Mischereinheit ein verdünntes mit dem Anästhesiemittel angereicherten Gasgemisch bereitzustellen.

Typischerweise erfolgt die Dosierung hierbei über eine besonders präzise Steuerung des Anästhesiemittelverdunsters.

Zur Kontrolle dieser Dosierung wird in US 5,967,141 vorgeschlagen, die Anästhesiemittelkonzentration an einem Auslass der Anästhesiemitteldosiervorrichtung zu bestimmen und die Dosierung durch eine Steuerung des Gasflusses durch die Gaszweige zu verändern, falls die bestimmte Anästhesiemittelkonzentration nicht mit einer vorbestimmten Sollkonzentration übereinstimmt. Weitere Dokumente im Stand der Technik sind DE 41 05 971 A1 sowie EP 0722 748 A2.

Aufgabe der vorliegenden Erfindung ist es, ein verbessertes Einstellen der Anästhesiemittelkonzentration, insbesondere ein besonders einfaches und leicht zu automatisierendes Einstellen der Anästhesiemittelkonzentration, zu ermöglichen.

Erfindungsgemäß wird zur Lösung dieser Aufgabe eine Anästhesiemitteldosiervorrichtung zum Einstellen einer Anästhesiemittelkonzentration in einem einem Anästhesieatemkreis bereitzustellenden Gasgemisch vorgeschlagen, mit einem ersten Gaszweig, einem zweiten Gaszweig, einem Anästhesiemittelverdunster, einer Mischereinheit, einer ersten Gasflussmesseinheit, einem Proportionalventil, einer Druckmesseinheit und einer Steuereinheit.

Der erste Gaszweig ist mit einer Atemgaszufuhr, insbesondere einer druckbeaufschlagten Atemgaszufuhr, verbindbar und ist ausgebildet, ein Atemgas durch den Anästhesiemittelverdunster zu der Mischereinheit zu führen.

Der zweite Gaszweig ist mit der Atemgaszufuhr, insbesondere der druckbeaufschlagten Atemgaszufuhr, verbindbar und ist ausgebildet, das Atemgas direkt zu der Mischereinheit zu führen.

Der Anästhesiemittelverdunster ist angeordnet und ausgebildet, das Atemgas in dem ersten Gaszweig mit einem Anästhesiemittel, insbesondere mit einem volatilen Anästhesiemittel, anzureichern.

Die Mischereinheit ist angeordnet und ausgebildet, das mit dem Anästhesiemittel angereicherte Atemgas aus dem ersten Gaszweig mit dem Atemgas aus dem zweiten Gaszweig zu mischen und das dadurch entstandene Gasgemisch einem Auslass der Anästhesiemitteldosiervorrichtung bereitzustellen.

Die erste Gasflussmesseinheit ist ausgebildet, einen ersten Gasfluss zu messen und ein entsprechendes erstes Gasflusssignal auszugeben.

Das Proportionalventil ist in dem zweiten Gaszweig angeordnet und dazu ausgebildet, ein Ventilsteuersignal zu empfangen und entsprechend dem Ventilsteuersignal eine Ventilöffnung einzustellen.

Die Druckmesseinheit ist angeordnet und ausgebildet, einen Gasdruck innerhalb des zweiten Gaszweigs zu messen und ein entsprechendes Ventildrucksignal auszugeben.

Die Steuereinheit ist ausgebildet, das Ventildrucksignal, das erste Gasflusssignal und ein zweites Gasflusssignal zu empfangen und abhängig von dem ersten und zweiten Gasflusssignal einen ersten Gaszweigfluss in dem ersten Gaszweig und einen zweiten Gaszweigfluss in dem zweiten Gaszweig zu bestimmen. Weiterhin ist die Steuereinheit ausgebildet, ein Sollkonzentrationssignal zu empfangen, das eine vorbestimmte Sollkonzentration des Anästhesiemittels am Auslass indiziert, und basierend auf der vorbestimmten Sollkonzentration und dem ersten und zweiten Gaszweigfluss einen Sollkammerdruck einer Verdunsterkammer des Anästhesiemittelverdunsters zu bestimmen. Schließlich ist die Steuereinheit weiterhin ausgebildet, abhängig von dem Sollkammerdruck und dem Ventildrucksignal eine Sollventilöffnung des Proportionalventils zu bestimmen, wobei die Sollventilöffnung derart bestimmt ist, dass durch die Sollventilöffnung des Proportionalventils in dem zweiten Gaszweig der Sollkammerdruck innerhalb der Anästhesiemitteldosiervorrichtung in dem ersten Gaszweig erreicht wird. Weiterhin ist die Steuereinheit ausgebildet, das die Sollventilöffnung indizierende Ventilsteuersignal an das Proportionalventil auszugeben.

Im Rahmen der Erfindung wurde erkannt, dass über ein Proportionalventil in dem zweiten Gaszweig, also dem Bypass-Zweig, der Kammerdruck innerhalb der Verdunsterkammer des Anästhesiemittelverdunsters eingestellt und dadurch die resultierende Anästhesiemittelkonzentration gesteuert werden kann. Weiterhin wurde erkannt, dass durch eine derartige Steuerung eines Proportionalventils eine aufwändige separate Flusssteuerung zwischen dem ersten und zweiten Gaszweig vermieden werden kann.

Die erfindungsgemäße Anästhesiemitteldosiervorrichtung stellt vorteilhaft die vorbestimmte Sollkonzentration des Anästhesiemittels über die Beeinflussung des Gasdrucks innerhalb des ersten und zweiten Gaszweigs sicher. Hierdurch können für das Einstellen der Anästhesiemittelkonzentration besonders robuste und kostengünstige Teile, wie beispielsweise das Proportionalventil oder die Druckmesseinheit, verwendet werden.

Vorteilhaft müssen in der erfindungsgemäßen Anästhesiemitteldosiervorrichtung nur wenige Teile gegenüber bekannten und bereits am Markt erhältlichen Dosiervorrichtungen verändert oder angepasst werden.

Weiterhin erlaubt das regelmäßige Messen des Gasdrucks innerhalb des zweiten Gaszweigs ein schnelles Erkennen bekannter Störquellen, wie etwa einer Leckage. Weiterhin erlaubt die Überwachung des Gasdrucks auch das Sicherstellen eines Mindest- und/oder eines Maximaldrucks innerhalb der Dosiervorrichtung, um beispielsweise das druckbeaufschlagte zugeführte Atemgas kontrolliert durch die beiden Gaszweige zu führen.

Ein weiterer Vorteil der erfindungsgemäßen Anästhesiemitteldosiervorrichtung liegt darin, dass ein besonders großer Bereich an unterschiedlichen vorbestimmten Sollkonzentrationen des Anästhesiemittels über das Proportionalventil eingestellt werden kann. Insbesondere kann trotz eines leichten Anästhesiemittelverdunsters eine hohe Abgabekonzentration des Anästhesiemittels bereitgestellt werden, da eine Konzentrationsveränderung ohne Beeinflussung der Temperatur der Verdunsterkammer möglich ist. Daher muss die Temperatur nicht aufwendig durch eine große Masse der Verdunsterkammer konstant gehalten werden. Vorzugsweise ist die in der Verdunsterkammer vorliegende Temperatur eine vorbestimmte Temperatur. Weiterhin kann die Verdunsterkammer einen Temperatursensor aufweisen, der ausgebildet ist, ein die Temperatur indizierendes Temperatursignal an die Steuereinheit auszugeben. Erfindungsgemäß verwendet die Steuereinheit die Temperatur der Verdunsterkammer, um den Sollkammerdruck in der Verdunsterkammer und/oder die Sollventilöffnung zu bestimmen.

Der erfindungsgemäße Ansatz, die Anästhesiemittelkonzentration über den Gasdruck innerhalb des ersten Gaszweigs einzustellen, basiert darauf, dass durch den Gasdruck innerhalb des ersten Gaszweigs der Stoffmengenanteil innerhalb der Verdunsterkammer und damit die Abgabekonzentration des Anästhesiemittels gezielt beeinflusst wird. Da der Partialdruck einer volatilen Flüssigkeit im Wesentlichen nur von der Temperatur abhängt, liegt dieser mit einer vorbestimmten und/oder bekannten Verdunsterkammertemperatur fest. Erfindungsgemäß wird jedoch ein Absolutdruck innerhalb der Verdunsterkammer entsprechend des Sollkammerdrucks verändert. Beispielsweise geht mit einem Anstieg dieses Absolutdrucks um den Faktor zwei bei konstanter Temperatur eine Verringerung des Anteils des Anästhesiemitteldampfes innerhalb der Verdunsterkammer um den Faktor zwei einher, so dass über die Steuerung des Absolutdrucks innerhalb der Verdunsterkammer die Anästhesiemittelkonzentration eingestellt werden kann.

Vorteilhaft können für die erfindungsgemäße Anästhesiemitteldosiervorrichtung verschiedene bekannte volatile Anästhesiemittel, wie beispielsweise Isofluran, Sevofluran und Desfluran, verwendet werden.

Die vorbestimmte Sollkonzentration wird beispielsweise durch eine manuelle Eingabe oder durch ein an den Anästhesieatemkreis angeschlossenes Anästhesiegerät vorgegeben.

Die Druckmesseinheit kann beispielsweise ein Differenzdruckmessgerät sein. Weiterhin kann die Druckmesseinheit Teil eines Druckmesssystems der erfindungsgemäßen Anästhesiemitteldosiervorrichtung sein.

Die Steuereinheit kann räumlich getrennt von den weiteren Teilen der Anästhesiemitteldosiervorrichtung angeordnet sein. Insbesondere kann die Steuereinheit einen Prozessor eines Medizingeräts umfassen. Die Steuereinheit kann weiterhin aus verschiedenen Steuermodulen, insbesondere aus räumlich getrennten Steuermodulen, bestehen. Sämtliche Teile der erfindungsgemäßen Anästhesiemitteldosiervorrichtung können in einem gemeinsamen Gehäuse oder zumindest teilweise auch beabstandet voneinander angeordnet sein.

Insbesondere kann die erfindungsgemäße Anästhesiemitteldosiervorrichtung separat von einem Anästhesiegerät zum Betreiben des Anästhesieatemkreises verwendet und angeordnet werden.

Vorzugsweise wird die Anästhesiemitteldosiervorrichtung ergänzt um zusätzliche Sensorik, die prüft, ob am Auslass ein Gasgemisch mit der vorbestimmten Sollkonzentration vorliegt.

Erfindungsgemäß ist die Sollventilöffnung des Proportionalventils eine Stellung des Proportionalventils, die angibt, wie viel Gas das Proportionalventil passieren kann. Das Ventilsteuersignal kann beispielsweise die konkrete Sollventilöffnung oder eine Veränderung, insbesondere eine inkrementelle Veränderung, der aktuell vorliegenden Ventilöffnung anzeigen. So kann erfindungsgemäß eine Abfolge einer Mehrzahl von Ventilsteuersignalen notwendig sein, um eine bestimmte Sollventilöffnung des Proportionalventils zu erreichen, insbesondere graduell zu erreichen. Erfindungsgemäß kann das Anzeigen der Sollventilöffnung beispielsweise über das Anzeigen eines einzustellenden Sollventileffekts erfolgen, wie etwa einen Soll-Druckabfall und/oder einen Soll-Volumenstrom und/oder einen Soll-Massenstrom. In einigen erfindungsgemäßen Ausführungsformen wird die Öffnung des Proportionalventils, also die Position eines beweglichen Teils des Proportionalventils nicht direkt bestimmt und vorgegeben, sondern die durch das bewegliche Teil entstehende Durchflussöffnung für das Gas wird über das Einstellen des Soll-Druckabfalls, des Soll-Volumenstroms und/oder des Soll-Massenstroms eingestellt und dadurch wird eine Sollventilöffnung indirekt angezeigt oder vorgegeben.

Ein Ventil ist im Rahmen dieser Erfindung allgemein ein Durchflussregelorgan, welches einen Durchfluss eines Fluids über die Position eines beweglichen Teils des Ventils und eine entsprechende Regelung der Durchflussöffnung regelt.

Der Aufbau eines Anästhesiemittelverdunsters, einer Mischereinheit und eines Proportionalventils sind dem Fachmann grundsätzlich bekannt, so dass dieser im Folgenden nicht im Detail erläutert wird. Weiterhin sind verschiedene Realisierungen einer Gasflussmesseinheit und einer Druckmesseinheit dem Fachmann auf diesem Gebiet ebenfalls bekannt, so dass mögliche bekannte Strukturen für derartige Messeinheiten ebenfalls im Folgenden nicht im Detail erläutert werden.

Das Bestimmen eines Wertes basierend auf vorbestimmten Informationen ist im Rahmen dieser Erfindung ein Bestimmen des Wertes durch unmittelbare Verwendung der vorbestimmten Informationen und/oder durch Verwendung von Informationen, die aufgrund der vorbestimmten Informationen zur Verfügung stehen. Das Bestimmen basierend und/oder abhängig von vorbestimmten Informationen kann auch unter Verwendung weiterer Informationen bei dieser Bestimmung erfolgen.

Nachfolgend werden bevorzugte Ausführungsformen des erfindungsgemäßen Systems beschrieben.

In einer besonders bevorzugten Ausführungsform weist die erfindungsgemäße Anästhesiemitteldosiervorrichtung weiterhin eine zweite Gasflussmesseinheit auf, die ausgebildet ist, einen zweiten Gasfluss zu bestimmen und basierend auf dem zweiten Gasfluss das zweite Gasflusssignal auszugeben. In dieser Ausführungsform kann durch die beiden Gasflussmesseinheiten direkt auf ein Verhältnis zwischen dem Gasfluss im ersten Gaszweig und dem Gasfluss im zweiten Gaszweig geschlossen werden. Weiterhin wird in dieser Ausführungsform vorzugsweise das zweite Gasflusssignal zumindest in regelmäßigen Abständen gemessen, insbesondere kontinuierlich gemessen. In einer alternativen Ausführungsform ist die Anästhesiemitteldosiervorrichtung dazu ausgebildet, das zweite Gasflusssignal von einem externen Gerät zu empfangen und/oder ein vorbestimmtes zweites Gasflusssignal für die Verarbeitung durch die Steuereinheit zu verwenden.

In einer bevorzugten Variante der vorhergehenden Ausführungsform ist die erste Gasflussmesseinheit in dem ersten Gaszweig und die zweite Gasflussmesseinheit in dem zweiten Gaszweig angeordnet. In dieser Variante können vorzugsweise direkt der Fluss in dem ersten Gaszweig und der Fluss in dem zweiten Gaszweig gemessen werden, um aus dem daraus resultierenden Verhältnis der beiden Flüsse untereinander und der vorbestimmten Sollkonzentration auf den Sollkammerdruck innerhalb der Verdunsterkammer zu schließen. Weiterhin reduziert die direkte Messung der beiden Flüsse gemäß dieser Variante die Gefahr von Messfehlern, die beispielsweise bei einer indirekten Messung mindestens einer der beiden Flüsse bestehen würde. So wird in einer alternativen Variante dieser Ausführungsform eine der beiden Gasflussmesseinheiten im Bereich der Atemgaszufuhr angeordnet, so dass durch einen bekannten Fluss innerhalb eines Gaszweigs und den gesamten Fluss im Bereich der Atemgaszufuhr auch der Fluss in dem entsprechenden anderen Gaszweig bestimmt werden kann.

In einer weiteren Ausführungsform umfasst die erste Gasflussmesseinheit und/oder die zweite Gasflussmesseinheit einen Differenzdrucksensor. Ein Differenzdrucksensor ist ein bekannter und in großen Mengen produzierter Sensortyp, so dass er kostengünstig verwendet werden kann. Der Aufbau eines solchen Differenzdrucksensors ist allgemein bekannt, so dass er im Folgenden nicht detailliert erläutert wird. Die Verwendung eines Differenzdrucksensors ist verglichen mit einem Sensor, der lediglich an einer Position einen einzigen Druck misst, besonders genau. In einer Variante diese Ausführungsform ist die Druckmesseinheit innerhalb des zweiten Gaszweigs ein Teil der zweiten Gasflussmesseinheit, die durch einen Differenzdrucksensor innerhalb des zweiten Gaszweigs gebildet ist.

In einer weiteren bevorzugten Ausführungsform ist die Druckmesseinheit relativ zur Flussrichtung zumindest teilweise vor dem Proportionalventil angeordnet. Eine derartige Anordnung der Druckmesseinheit ermöglicht eine besonders präzise Bestimmung des durch das Proportionalventil verursachten Druckes innerhalb des zweiten Gaszweigs.

In einer vorteilhaften Variante der vorhergehenden Ausführungsform ist die Druckmesseinheit weiterhin ausgebildet, relativ zur Flussrichtung hinter dem Proportionalventil den Gasdruck innerhalb des zweiten Gaszweigs zu messen. In dieser Ausführungsform kann besonders präzise die Wirkung des Proportionalventils auf den Gasdruck innerhalb des zweiten Gaszweigs überprüft werden. Es kann besonders präzise der durch das Proportionalventil verursachte Druckabfall ermittelt und für die weitere Verrechnung bei der Bestimmung des Sollkammerdrucks und/oder der Sollventilöffnung verwendet werden. Der durch das Proportionalventil verursachte Druckabfall liegt vorzugsweise in einem Bereich zwischen 200 mbar und 2000mbar.

In einer besonders bevorzugten Ausführungsform weist die erfindungsgemäße Anästhesiemitteldosiervorrichtung ein weiteres Ventil auf, das in dem ersten Gaszweig relativ zur Flussrichtung hinter dem Anästhesiemittelverdunster angeordnet ist und das ausgebildet ist, ein weiteres Ventilsteuersignal zu empfangen und entsprechend des weiteren Ventilsteuersignals eine weitere Ventilöffnung einzustellen. Durch die Steuerung des Proportionalventils in dem zweiten Gaszweig und des weiteren Ventils in dem ersten Gaszweig, kann besonders effektiv der Sollkammerdruck innerhalb der Verdunsterkammer über die Steuerung des Gasdrucks innerhalb der zwei Gaszweige erreicht werden. Durch das weitere Ventil können die Flüsse in den beiden Gaszweigen separat eingestellt werden, so dass jedes beliebige Verhältnis zwischen den beiden Flüssen eingestellt werden kann. Hierdurch ergibt sich ein Freiheitsgrad hinsichtlich des Absolutdrucks in der Verdampferkammer, der somit nahezu beliebig eingestellt werden kann.

Das weitere Ventil ist in einer erfindungsgemäßen Variante ein Schaltventil, das in einem Puls-Pause-Modus angesteuert wird, und in einer anderen Variante ein durch einen mittels Druckbeaufschlagung biegbaren Kunststoff realisiertes Ventil. In einer besonders bevorzugten Variante der vorhergehenden Ausführungsform handelt es sich bei dem weiteren Ventil um ein Proportionalventil. Ein Proportionalventil kann besonders einfach und kostengünstig zur Steuerung des Gasdrucks innerhalb des ersten Gaszweigs verwendet werden.

In einer weiteren besonders bevorzugten Ausführungsform ist das Ventilsteuersignal derart bereitgestellt, dass ein vorbestimmter Mindestdruck innerhalb der Verdunsterkammer der Anästhesiemitteldosiervorrichtung nicht unterschritten wird. Durch das Bereitstellen eines Mindestdruckes, kann sichergestellt werden, dass die typischerweise vorliegenden Druckschwankungen im nachgeschalteten Atemkreis keinen oder lediglich einen geringen Einfluss auf die Genauigkeit der eingestellten Anästhesiem ittelkonzentration am Auslass der Anästhesiemitteldosierungsvorrichtung hat. So würde ein zu geringer Druck innerhalb der beiden Gaszweige dazu führen, dass die Druckschwankungen im Atemkreis die Durchflusskonstanz im Anästhesiemittelverdunster stören. In einer bevorzugten Variante dieser Ausführungsform ist das Ventilsteuersignal derart bereitgestellt, dass der vorbestimmte Mindestdruck mindestens 200 mbar, insbesondere mindestens 300 mbar beträgt.

In einer bevorzugten Ausführungsform weist die Anästhesiemitteldosiervorrichtung weiterhin eine zwischen Mischereinheit und Auslass angeordnete Konzentrationsmesseinheit auf, die ausgebildet ist, eine aktuelle Konzentration des Anästhesiemittels zu bestimmen und ein entsprechendes Konzentrationssignal auszugeben, wobei die Steuereinheit weiterhin ausgebildet ist, das Konzentrationssignal zu empfangen und die Sollventilöffnung abhängig von der durch das Konzentrationssignal indizierten aktuellen Konzentration zu bestimmen. In dieser Ausführungsform wird besonders zuverlässig sichergestellt, dass die Anästhesiemittelkonzentration an dem Auslass der Anästhesiemitteldosiervorrichtung der vorbestimmten Sollkonzentration des Anästhesiemittels entspricht. So kann bei der Steuerung der Sollventilöffnung des Proportionalventils neben dem anhand der Flüsse in den Gaszweigen abgeleiteten Absolutdruck innerhalb der Verdunsterkammer auch die tatsächlich vorliegende Anästhesiemittelkonzentration am Auslass berücksichtigt werden. In einer besonders bevorzugten Variante dieser Ausführungsform weist die Anästhesiemitteldosiervorrichtung weiterhin das weitere Ventil in dem ersten Gaszweig auf. Hierdurch kann neben der Sensorik am Auslass auch eine besonders zuverlässige Aktorik bereitgestellt werden, durch die festgestellte Abweichungen bei der Anästhesiemittelkonzentration korrigiert werden können.

In einer weiteren bevorzugten Ausführungsform weist die Anästhesiemitteldosiervorrichtung weiterhin eine weitere Druckmesseinheit auf, die ausgebildet ist, relativ zur Flussrichtung hinter dem Anästhesiemittelverdunster den Gasdruck innerhalb des ersten Gaszweigs zu messen. Hierdurch kann der Absolutdruck des mit dem Anästhesiemittel angereicherten Atemgases bestimmt werden, wodurch besonders genau eine aktuelle Konzentration des Anästhesiemittels in dem Atemgas über bereits bekannte Verfahren bestimmt werden kann.

In einer weiteren bevorzugten Variante der vorhergehenden Ausführungsform weist die Konzentrationsmesseinheit mindestens ein Konzentrationsmessmodul auf, das ausgebildet ist, die Konzentration des Anästhesiemittels in dem Gasgemisch basierend auf einer Wärmeleitfähigkeitsmessung und/oder einer elektrochemischen Messung zu bestimmen. Hierbei ist die Konzentrationsmesseinheit vorzugsweise dazu ausgebildet, im Rahmen der Wärmeleitfähigkeitsmessung und/oder der elektrochemischen Messung sowohl einen Messwert, insbesondere eine Temperatur, im Bereich des Auslasses, als auch einen Messwert, insbesondere eine Temperatur, im Bereich des zweiten Gaszweigs zu bestimmen. Anhand einer derartigen Vergleichsmessung kann beispielsweise unter Ausnutzung der bekannten Wärmeleitfähigkeit gängiger als Atemgas genutzter Gasgemische, wie etwa eines Sauerstoff-StickstoffGemisches und/oder eines Sauerstoff-Lachgas-Gemisches, auf die Anästhesiemittelkonzentration geschlossen werden. In dieser Variante kann vorteilhaft ein besonders kostengünstiger und einfach bereitzustellender Wärmeleitfähigkeitssensor verwendet werden.

In einer weiteren bevorzugten Variante der vorhergehenden Ausführungsform ist die Steuereinheit ausgebildet, die Sollventilöffnung abhängig von einer Differenz zwischen aktueller Konzentration und vorbestimmter Sollkonzentration zu bestimmen. In dieser Variante wird vorteilhaft die durch die Konzentrationsmesseinheit zusätzlich erhaltene Information bezüglich der aktuell vorliegenden Anästhesiemittelkonzentration am Auslass für das besonders präzise Einstellen des Proportionalventils verwendet.

In einer weiteren Ausführungsform ist die Steuereinheit ausgebildet, ein Frischgassignal zu empfangen, das einen vorbestimmten an dem Auslass bereitzustellenden Frischgasfluss des Gasgemisches indiziert, und wobei die Steuereinheit weiterhin ausgebildet ist, die Sollventilöffnung und/oder den Sollkammerdruck abhängig von dem vorbestimmten Frischgasfluss zu bestimmen. Frischgas ist hierbei die Bezeichnung für das durch die Anästhesiemitteldosiervorrichtung dem Anästhesieatemkreis beigefügte Gasgemisch. In einer besonders bevorzugten Variante dieser Ausführungsform weist die Anästhesiemitteldosiervorrichtung weiterhin das weitere Ventil, insbesondere das weitere Proportionalventil, in dem ersten Gaszweig auf. In dieser Variante kann besonders direkt der Atemgasfluss durch die beiden Ventile in dem ersten und zweiten Gaszweig entsprechend des bereitzustellenden Frischgasflusses eingestellt werden. Beispielsweise kann der Frischgasfluss am Auslass bei gleichbleibender Anästhesiemittelkonzentration dadurch erhöht werden, dass der Anteil an Frischgas aus dem zweiten Gaszweig reduziert wird, so dass auch eine durch den erhöhten Fluss reduzierte Aufnahme von Anästhesiemittel in dem ersten Gaszweig zu einer nach dem Passieren der Mischereinheit gleich bleibenden Anästhesiemittelkonzentration des entstandenen Gasgemisches führt. In diesem Szenario werden sowohl das Proportionalventil in dem zweiten Gaszweig, als auch das weitere Ventil in dem ersten Gaszweig verwendet, um den vorbestimmten Frischgasfluss bereitzustellen. Die Bereitstellung von verschiedenen Werten für den Frischgasfluss kann vorzugsweise in einem Bereich zwischen 0,5 l/min und 20 l/min eingestellt werden. Die einstellbaren Konzentrationsbereiche sind abhängig von dem verwendeten Anästhesiemittel.

In einer weiteren Ausführungsform ist mindestens eines der Proportionalventile dazu ausgebildet, ein Ventilöffnungssignal auszugeben, das die aktuell vorliegende Ventilöffnung des betreffenden Proportionalventils indiziert. Vorzugsweise ist die Steuereinheit in einer Variante dieses Ausführungsbeispiels dazu ausgebildet, das Ventilöffnungssignal zu empfangen und beim Erzeugen des Ventilsteuersignals zu verwenden.

In einer weiteren besonders bevorzugten Ausführungsform weist der Anästhesiemittelverdunster weiterhin eine Temperaturmesseinheit auf, die ausgebildet ist eine Temperatur innerhalb der Verdunsterkammer des Anästhesiemittelverdunsters zu bestimmen und ein Temperatursignal auszugeben, das die innerhalb der Verdunsterkammer vorliegende Temperatur indiziert. Vorzugsweise ist die Steuereinheit in einer Variante dieser Ausführungsform dazu ausgebildet, das Temperatursignal zu empfangen und den Sollkammerdruck und/oder die Sollventilöffnung weiterhin abhängig von dem Temperatursignal zu bestimmen. Hierdurch kann vorteilhaft auf eine Veränderung der Temperatur innerhalb der Verdunsterkammer und eine dadurch entstehende Veränderung des Verhältnisses von Partialdruck des Anästhesiemittels und Absolutdruck in der Verdunsterkammer reagiert werden. Dies ermöglicht eine besonders präzise Bereitstellung der vorbestimmten Sollkonzentration des Anästhesiemittels. In einer alternativen Ausführungsform ist der Anästhesiemittelverdunster ausgebildet, eine konstante vorbestimmte Temperatur innerhalb der Verdunsterkammer bereitzustellen.

Gemäß einem weiteren Aspekt der Erfindung wird zur Lösung der oben genannten Aufgabe ein Verfahren zum Einstellen einer Anästhesiemittelkonzentration in einem einem Anästhesieatemkreis bereitzustellenden Gasgemisch vorgeschlagen. Das Verfahren weist die folgenden Schritte auf:
- Bereitstellen eines ersten und zweiten Gaszweigs, wobei der erste Gaszweig mit einer Atemgaszufuhr verbindbar ist und ausgebildet ist, ein Atemgas durch einen Anästhesiemittelverdunster zu einer Mischereinheit zu führen, und wobei der zweite Gaszweig mit der Atemgaszufuhr verbindbar ist und ausgebildet ist, das Atemgas direkt zu der Mischereinheit zu führen, und wobei die Mischereinheit, angeordnet und ausgebildet ist, das mit einem Anästhesiemittel angereicherte Atemgas aus dem ersten Gaszweig mit dem Atemgas aus dem zweiten Gaszweig zu mischen und das dadurch entstandene Gasgemisch einem Auslass der Anästhesiemitteldosiervorrichtung bereitzustellen;
- Empfangen eines Ventilsteuersignals und Einstellen einer Ventilöffnung eines Proportionalventils entsprechend dem Ventilsteuersignal;
- Messen eines ersten Gasflusses und Ausgeben eines entsprechenden ersten Gasflusssignals;
- Messen eines Gasdrucks innerhalb des zweiten Gaszweigs und Ausgeben eines entsprechenden Ventildrucksignals;
- Empfangen des ersten Gasflusssignals und eines zweiten Gasflusssignals und Bestimmen eines ersten Gaszweigflusses in dem ersten Gaszweig und eines zweiten Gaszweigflusses in dem zweiten Gaszweig abhängig von dem ersten und zweiten Gasflusssignal;
- Empfangen eines Sollkonzentrationssignals, das eine vorbestimmte Sollkonzentration des Anästhesiemittels am Auslass indiziert, und Bestimmen eines Sollkammerdrucks einer Verdunsterkammer des Anästhesiemittelverdunsters basierend zumindest auf der vorbestimmten Sollkonzentration und dem ersten und zweiten Gaszweigfluss;
- Bestimmen einer Sollventilöffnung des Proportionalventils abhängig von dem Sollkammerdruck und dem Ventildrucksignal, wobei die Sollventilöffnung derart bestimmt wird, dass durch die Sollventilöffnung des Proportionalventils in dem zweiten Gaszweig der Sollkammerdruck innerhalb des Anästhesiemittelverdunsters in dem ersten Gaszweig erreicht wird;
- Ausgeben des die Sollventilöffnung indizierenden Ventilsteuersignals an das Proportionalventil.

Das Verfahren gemäß dem weiteren Aspekt der Erfindung ermöglicht vorteilhaft eine besonders einfache Steuerung der dem Anästhesieatemkreis bereitgestellten Anästhesiemittelkonzentration. Weiterhin kann das erfindungsgemäße Verfahren besonders einfach automatisiert werden. So kann abhängig von der vorbestimmten Sollkonzentration durch die Regelung des Proportionalventils über den Sollkammerdruck innerhalb der Verdunsterkammer und daher über den Absolutdruck innerhalb der Verdunsterkammer die Anästhesiemittelkonzentration innerhalb des ersten Gaszweigs zuverlässig eingestellt werden.

Das erfindungsgemäße Verfahren ist ein iteratives Verfahren. Während der erste Schritt des Bereitstellens von Teilen typischerweise bei der Herstellung der Anästhesiemitteldosiervorrichtung stattfindet, wird die Ventilöffnung des Proportionalventils in regelmäßigen Abständen iterativ eingestellt. So wird nach einer Einstellung der Ventilöffnung weiterhin der Gasdruck innerhalb des zweiten Gaszweigs gemessen, wodurch weiterhin abhängig von den aktuellen Messwerten innerhalb der Anästhesiemitteldosiervorrichtung der Sollkammerdruck, und abhängig vom Sollkammerdruck die Sollventilöffnung bestimmt und als neues Ventilsteuersignal ausgegeben wird.

In einer besonders vorteilhaften Ausführungsform des erfindungsgemäßen Verfahrens umfasst das Verfahren weiterhin die Schritte
- Bereitstellen eines weiteren Ventils, das in dem ersten Gaszweig relativ zur Flussrichtung hinter dem Anästhesiemittelverdunster angeordnet ist;
- Empfangen eines weiteren Ventilsteuersignals und Einstellen einer weiteren Ventilöffnung des weiteren Ventils entsprechend des weiteren Ventilsteuersignals.

Durch das Einstellen des Proportionalventils in dem zweiten Gaszweig und des weiteren Ventils einem ersten Gaszweig kann besonders zuverlässig der Sollkammerdruck innerhalb der Verdunsterkammer des Anästhesiemittelverdunsters ist eingestellt werden.

In einer weiteren besonders bevorzugten Ausführungsform des erfindungsgemäßen Verfahrens weist der Anästhesiemittelverdunster weiterhin eine Temperaturmesseinheit auf, die ausgebildet ist eine Temperatur innerhalb der Verdunsterkammer des Anästhesiemittelverdunsters zu bestimmen und ein Temperatursignal auszugeben, dass die innerhalb der Verdunsterkammer vorliegende Temperatur indiziert. Vorzugsweise umfasst das Verfahren dabei weiterhin die Schritte
- Empfangen des Temperatursignals;
- Bestimmen des Sollkammerdrucks weiterhin abhängig von dem Temperatursignal.

Die Erfindung soll nun anhand von in den Figuren schematisch dargestellten, vorteilhaften Ausführungsbeispielen näher erläutert werden. Von diesen zeigen im Einzelnen:
- Fig. 1: eine schematische Darstellung eines ersten Ausführungsbeispiels einer erfindungsgemäßen Anästhesiemitteldosiervorrichtung;
- Fig. 2: eine schematische Darstellung eines zweiten Ausführungsbeispiels der erfindungsgemäßen Anästhesiemitteldosiervorrichtung;
- Fig. 3: eine schematische Darstellung eines dritten Ausführungsbeispiels der erfindungsgemäßen Anästhesiemitteldosiervorrichtung;
- Fig. 4: eine schematische Darstellung eines vierten Ausführungsbeispiels der erfindungsgemäßen Anästhesiemitteldosiervorrichtung;
- Fig. 5: eine schematische Darstellung eines fünften Ausführungsbeispiels der erfindungsgemäßen Anästhesiemitteldosiervorrichtung;
- Fig. 6: ein Ablaufdiagramm eines Ausführungsbeispiels eines Verfahrens gemäß dem weiteren Aspekt der Erfindung.

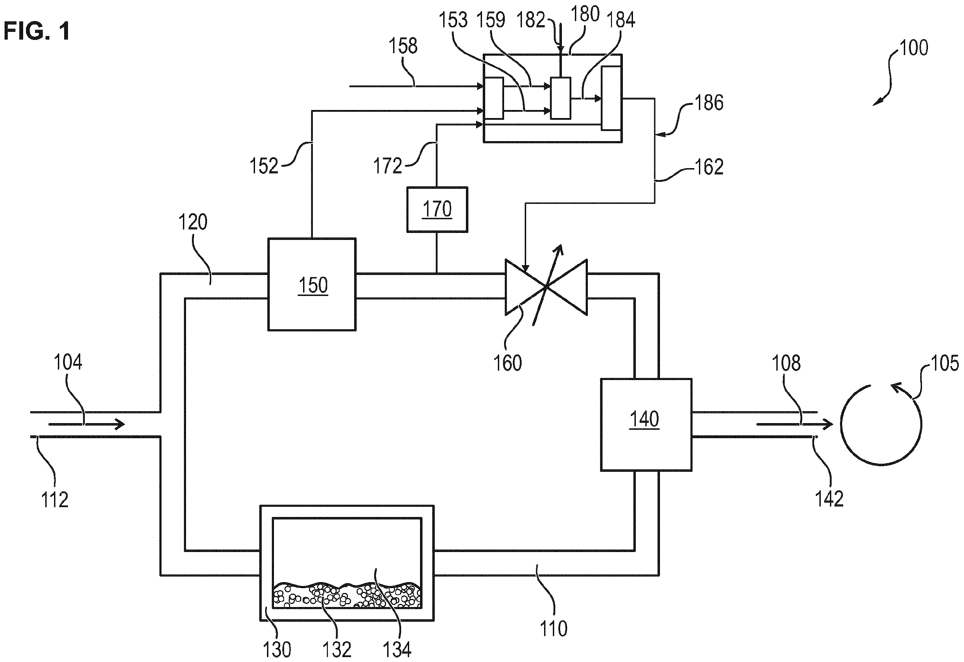

Fig. 1 zeigt eine schematische Darstellung eines ersten Ausführungsbeispiels einer erfindungsgemäßen Anästhesiemitteldosiervorrichtung 100.

Die Anästhesiemitteldosiervorrichtung 100 ist zum Einstellen einer Anästhesiemittelkonzentration ausgebildet in einem einem Anästhesieatemkreis 105 bereitzustellenden Gasgemisch 108, mit einem ersten Gaszweig 110, einem zweiten Gaszweig 120, einem Anästhesiemittelverdunster 130, einer Mischereinheit 140, einer ersten Gasflussmesseinheit 150, einem Proportionalventil 160, einer ersten Druckmesseinheit 170 und einer Steuereinheit 180.

Der erste Gaszweig 110 ist mit einer Atemgaszufuhr 112 verbindbar und ausgebildet, ein Atemgas 104 durch den Anästhesiemittelverdunster 130 zu der Mischereinheit 140 zu führen.

Der zweite Gaszweig 120 ist mit der Atemgaszufuhr verbindbar und ausgebildet, das Atemgas 104 direkt zu der Mischereinheit 140 zu führen. Die beiden Gaszweige 110, 120 weisen jeweils einen Hohlraum auf, durch den das Atemgas 104 jeweils geführt wird und der durch eine rohrförmige Wandung begrenzt ist.

Der Anästhesiemittelverdunster 130 ist angeordnet und ausgebildet, das Atemgas 104 in dem ersten Gaszweig 110 mit einem Anästhesiemittel 132 anzureichern. Das Anästhesiemittel 132 befindet sich dafür vorzugsweise in einer Verdunsterkammer 134. Typischerweise liegt das Anästhesiemittel 132 innerhalb der Verdunsterkammer 134 sowohl flüssig als auch gasförmig vor. Die Struktur einer derartigen Verdunsterkammer ist allgemein bekannt und wird daher im Folgenden nicht im Detail erläutert.

Die Mischereinheit 140 ist angeordnet und ausgebildet, das mit dem Anästhesiemittel 132 angereicherte Atemgas 104 aus dem ersten Gaszweig 110 mit dem Atemgas 104 aus dem zweiten Gaszweig 120 zu mischen und das dadurch entstandene Gasgemisch 108 einem Auslass 142 der Anästhesiemitteldosiervorrichtung 100 bereitzustellen. In dem dargestellten Ausführungsbeispiel ist die Mischereinheit 140 beabstandet von dem Auslass 142 angeordnet. In einem nicht dargestellten Ausführungsbeispiel umfasst die Mischereinheit den Auslass der Anästhesiemitteldosiervorrichtung.

Die erste Gasflussmesseinheit 150 ist ausgebildet, einen ersten Gasfluss zu messen und ein entsprechendes erstes Gasflusssignal 152 auszugeben. Vorliegend ist die erste Gasflussmesseinheit 150 in dem zweiten Gaszweig 120 angeordnet und misst direkt den Gasfluss durch den zweiten Gaszweig 120. Das erste Gasflusssignal 152 indiziert daher direkt den Gasfluss durch den zweiten Gaszweig 120. In dem dargestellten Ausführungsbeispiel handelt es sich bei der Gasflussmesseinheit 150 um einen Differenzdrucksensor mit einer Drosseleinheit. In einem nicht dargestellten Ausführungsbeispiel ist die erste Gasflussmesseinheit gebildet durch einen Ultraschall-Durchflusssensor, einen magnetisch-induktiven Durchflusssensor und/oder einen Durchflusssensor einer anderen bekannten Bauart.

Das Proportionalventil 160 ist in dem zweiten Gaszweig angeordnet und dazu ausgebildet, ein Ventilsteuersignal 162 zu empfangen und entsprechend dem Ventilsteuersignal 162 eine Ventilöffnung einzustellen. Der Aufbau eines derartigen Proportionalventils ist dem Fachmann auf diesem Gebiet bekannt und wird daher nicht im Folgenden detailliert erläutert.

Die Druckmesseinheit 170 ist angeordnet und ausgebildet, einen Gasdruck innerhalb des zweiten Gaszweigs 120 zu messen und ein entsprechendes Ventildrucksignal 172 auszugeben. Vorliegend ist die Druckmesseinheit 170 insbesondere dazu ausgebildet, den Gasdruck relativ zur Flussrichtung vor dem Proportionalventil 160 zu messen. Vorliegend handelt es sich bei der Druckmesseinheit 170 um einen Absolutdrucksensor, der einen vorliegenden Absolutdruck innerhalb des zweiten Gaszweigs 120 misst.

Die Steuereinheit 180 ist ausgebildet, das Ventildrucksignal 172, das erste Gasflusssignal 152 und ein zweites Gasflusssignal 158 zu empfangen und abhängig von dem ersten und zweiten Gasflusssignal 152, 158 einen ersten Gaszweigfluss 159 in dem ersten Gaszweig 110 und einen zweiten Gaszweigfluss 153 in dem zweiten Gaszweig 120 zu bestimmen. Das erste Gasflusssignal 152 indiziert bereits den zweiten Gaszweigfluss 153 in dem zweiten Gaszweig 120, da die erste Gasflussmesseinheit 150 in dem zweiten Gaszweig 120 angeordnet ist. In einem nicht dargestellten Ausführungsbeispiel ist die erste Gasflussmesseinheit in dem ersten Gaszweig oder im Bereich der Atemgaszufuhr angeordnet, so dass nur indirekt, beispielsweise durch ein Verhältnis zu einem weiteren gemessenen Gasfluss, auf den zweiten Gaszweigfluss in dem zweiten Gaszweig geschlossen werden kann. Das zweite Gasflusssignal 158 wird durch eine weitere in Fig. 1 nicht dargestellte Messeinheit bereitgestellt und/oder als vorbestimmtes, die Atemgaszufuhr charakterisierendes, zweites Gasflusssignal bereitgestellt. Weiterhin ist die Steuereinheit 180 ausgebildet, ein Sollkonzentrationssignal 182 zu empfangen, das eine vorbestimmte Sollkonzentration des Anästhesiemittels am Auslass 142 indiziert, und basierend auf der vorbestimmten Sollkonzentration und dem ersten und zweiten Gaszweigfluss 153, 159 einen Sollkammerdruck 184 der Verdunsterkammer 134 des Anästhesiemittelverdunsters 130 zu bestimmen. In dem dargestellten erfindungsgemäßen Ausführungsbeispiel erfolgt die Einstellung der Anästhesiemittelkonzentration an dem Auslass 142 entsprechend der vorbestimmten Sollkonzentration durch die Einstellung des Druckes innerhalb der Verdunsterkammer 134. Die Steuereinheit 180 ist hierbei weiterhin ausgebildet, abhängig von dem Sollkammerdruck 184 und dem Ventildrucksignal 172 eine Sollventilöffnung 186 des Proportionalventils 160 zu bestimmen, wobei die Sollventilöffnung 186 derart bestimmt ist, dass durch die Sollventilöffnung 186 des Proportionalventils 160 der Sollkammerdruck 184 innerhalb des Anästhesiemittelverdunsters 130 in dem ersten Gaszweig 110 erreicht wird. Über das Proportionalventil 160 staut sich also so viel oder so wenig Atemgas 104, dass der Absolutdruck innerhalb der Verdunsterkammer 134 dem bestimmten Sollkammerdruck 184 entspricht. Abhängig von dem Gasfluss durch die Verdunsterkammer 134 und der Temperatur innerhalb der Verdunsterkammer 134, die im dargestellten Ausführungsbeispiel vorzugsweise konstant auf einer vorbestimmten Temperatur gehalten wird, kann dadurch die Anästhesiemittelkonzentration des die Verdunsterkammer 134 verlassenden Gases eingestellt werden. Dadurch, dass die Konzentration des Anästhesiemittels nicht über die Temperatur der Verdunsterkammer 134, sondern über den Gasdruck des zugeführten Atemgases 104 eingestellt wird, ist ein besonders schnelles Verändern der Anästhesiemittelkonzentration möglich. Typischerweise kann der Gasdruck deutlich schneller und einfacher verändert und konstant gehalten werden als die Temperatur innerhalb der Verdunsterkammer 134.

Schließlich ist die Steuereinheit 180 weiterhin ausgebildet, das die Sollventilöffnung 186 indizierende Ventilsteuersignal 162 an das Proportionalventil 160 auszugeben. Über dieses Ventilsteuersignal 162 wird die Ventilöffnung des Proportionalventils 160 zumindest inkrementell, vorzugsweise direkt, auf die Sollventilöffnung 186 gebracht. Erfindungsgemäß wird für die dadurch entstandene Ventilöffnung weiterhin über die erste Gasflussmesseinheit 150 und die Druckmesseinheit 170 eine Steuerung des Proportionalventils 160 durch die Steuereinheit 180 ermöglicht. Sollte innerhalb der Verdunsterkammer 134 bereits der Sollkammerdruck 184 vorliegen, wird kein Ventilsteuersignal 162 ausgegeben oder es wird ein Ventilsteuersignal 162 ausgegeben, das die aktuelle Ventilöffnung als Sollventilöffnung 186 anzeigt.

Vorzugsweise ist die Steuereinheit 180 in dem dargestellten Ausführungsbeispiel dazu ausgebildet, das Ventilsteuersignal 162 derart bereitzustellen, dass ein vorbestimmter Mindestdruck innerhalb der Verdunsterkammer 134 des Anästhesiemittelverdunsters 130 nicht unterschritten wird. Der vorbestimmte Mindestdruck beträgt beispielsweise mindestens 200 mbar, insbesondere mindestens 300 mbar. In einer weiteren Ausführungsform ist der Mindestdruck innerhalb der Verdunsterkammer abhängig vom Mengenstrom des Atemgases 104.

In einem nicht dargestellten Ausführungsbeispiel ist eine weitere Druckmesseinheit in dem ersten Gaszweig angeordnet. Dabei ist die weitere Druckmesseinheit vorzugsweise relativ zur Flussrichtung hinter dem Anästhesiem ittelverdunster angeordnet.

Die verschiedenen Teile der erfindungsgemäßen Anästhesiemitteldosiervorrichtung 100 sind in dem dargestellten Ausführungsbeispiel in einem gemeinsamen Gehäuse (nicht dargestellt) angeordnet, welches an den Anästhesieatemkreis 105 angeschlossen werden kann. In einem anderen Ausführungsbeispiel ist die erfindungsgemäße Anästhesiem itteldosiervorrichtung in ein Anästhesiegerät mit einem entsprechenden Anästhesieatemkreis integriert. In einem weiteren Ausführungsbeispiel sind die verschiedenen Teile der erfindungsgemäßen Anästhesiemitteldosiervorrichtung zumindest teilweise in verschiedenen Gehäusen angeordnet.

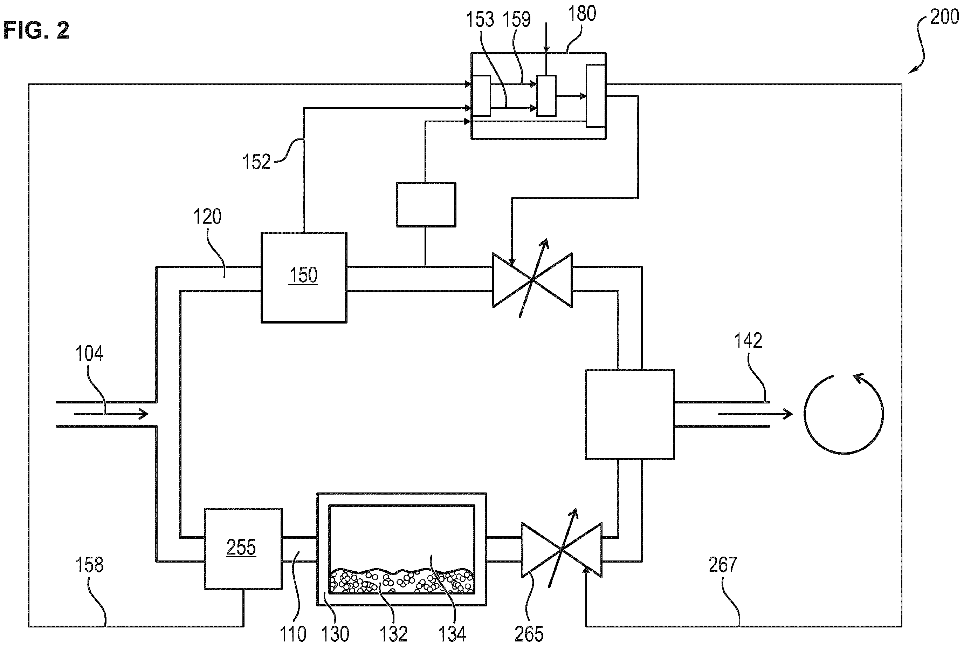

Fig. 2 zeigt eine schematische Darstellung eines zweiten Ausführungsbeispiels der erfindungsgemäßen Anästhesiemitteldosiervorrichtung 200.

Die Anästhesiemitteldosiervorrichtung 200 unterscheidet sich von der in Fig. 1 dargestellten Anästhesiem itteldosiervorrichtung 100 dadurch, dass sie weiterhin eine zweite Gasflussmesseinheit 255 aufweist, die ausgebildet ist, den Gasfluss innerhalb des ersten Gaszweigs 110 zu bestimmen und basierend auf diesem Gasfluss das zweite Gasflusssignal 158 auszugeben. Die zweite Gasflussmesseinheit 255 ist dabei als Differenzdrucksensor ausgebildet. In einem nicht dargestellten Ausführungsbeispiel ist die zweite Gasflussmesseinheit ein Ultraschall-Durchflusssensor oder ein magnetisch-induktiver Durchflusssensor. Durch die Anordnung der beiden Gasflussmesseinheiten 150, 255 werden direkt die Flüsse des Atemgases 104 innerhalb der beiden Gaszweige 110, 120 und an die Steuereinheit 180 ausgegeben. Das Bestimmen des ersten und zweiten Gaszweigflusses 153, 159 umfasst daher lediglich ein Auslesen der entsprechenden Flüsse aus dem ersten und zweiten Gasflusssignal 152, 158.

Weiterhin unterscheidet sich die Anästhesiemitteldosiervorrichtung 200 von der in Fig. 1 dargestellten Anästhesiemitteldosiervorrichtung 100 dadurch, dass ein weiteres Ventil 265 in dem ersten Gaszweig 110 angeordnet ist. Das weitere Ventil 265 befindet sich relativ zur Flussrichtung hinter dem Anästhesiemittelverdunster 130 und ist ausgebildet, ein weiteres Ventilsteuersignal 267 von der Steuereinheit 180 zu empfangen und entsprechend des weiteren Ventilsteuersignals 267 eine weitere Ventilöffnung einzustellen. Das weitere Ventil 265 ist in dem dargestellten Ausführungsbeispiel ein Proportionalventil. In einem nicht dargestellten Ausführungsbeispiel handelt es sich um ein Schaltventil, das im Puls-Pause-Modus angesteuert wird. Die weitere Ventilöffnung beschreibt die konkrete Stellung des weiteren Ventils 265, also einen Durchflussquerschnitt des durch das weitere Ventil 265 hindurchfließenden Atemgases 104.

Durch das weitere Ventil 265 kann in dem dargestellten Ausführungsbeispiel besonders vorteilhaft eine sehr genaue Einstellung der Druckverhältnisse innerhalb des ersten und zweiten Gaszweigs 110, 120 ermöglicht werden. Somit kann aufgrund der Steuerung der Anästhesiemittelkonzentration am Auslass über die Druckverhältnisse innerhalb der Verdunsterkammer 134 auch besonders präzise die Anästhesiemittelkonzentration am Auslass 142 eingestellt werden. Die Einstellung der Druckverhältnisse innerhalb der Verdunsterkammer 134 umfasst erfindungsgemäß das Einstellen des Absolutdrucks des zugeführten Atemgases 104 relativ zu dem über die Temperatur innerhalb der Verdunsterkammer 134 gegebenen Partialdruck des Anästhesiemittels 132.

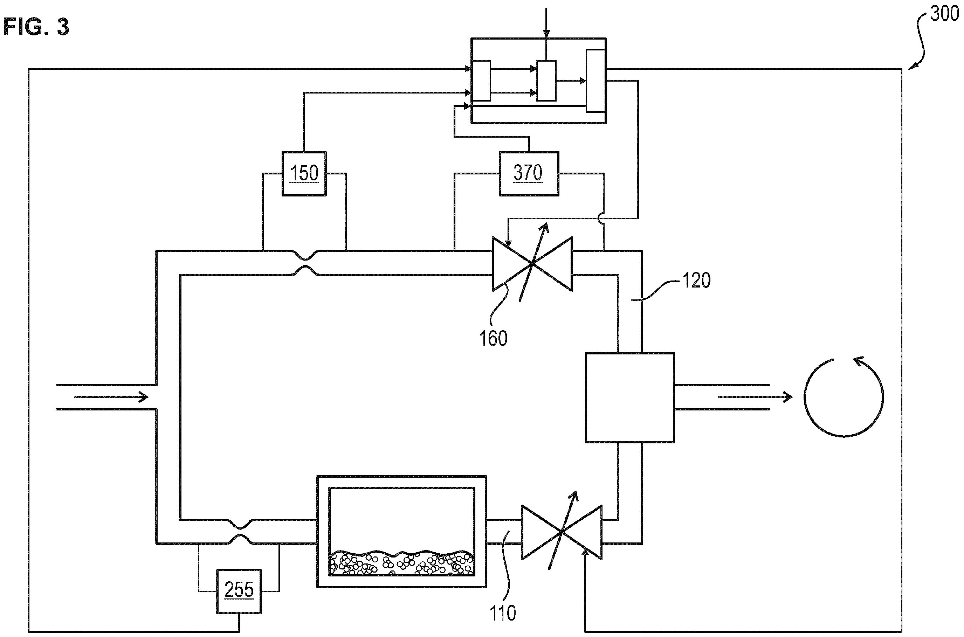

Fig. 3 zeigt eine schematische Darstellung eines dritten Ausführungsbeispiels der erfindungsgemäßen Anästhesiemitteldosiervorrichtung 300.

Die Anästhesiemitteldosiervorrichtung 300 unterscheidet sich von der in Fig. 2 dargestellten Anästhesiemitteldosiervorrichtung 200 dadurch, dass die Druckmesseinheit 370 weiterhin ausgebildet ist, relativ zur Flussrichtung hinter dem Proportionalventil 160 den Gasdruck innerhalb des zweiten Gaszweigs 120 zu messen. Hierdurch kann besonders präzise die über das Proportionalventil 160 bereitgestellte Druckdifferenz innerhalb des zweiten Gaszweigs 120 gemessen und/oder überwacht werden.

In einem nicht dargestellten vorteilhaften Ausführungsbeispielen ist eine weitere Druckmesseinheit im Bereich des weiteren Ventils im ersten Gaszweig angeordnet. In einer Variante dieses vorteilhaften Ausführungsbeispiels ist die weitere Druckmesseinheit relativ zur Flussrichtung sowohl vor als auch hinter dem weiteren Ventil angeordnet, so dass über diese Druckmesseinheit besonders präzise die durch das weitere Ventil bereitgestellte Druckdifferenz gemessen und/oder überwacht werden kann.

Schließlich ist im Rahmen des in Fig. 3 dargestellten Ausführungsbeispiels weiterhin eine Struktur der jeweils als Differenzdrucksensor ausgebildeten ersten und zweiten Gasflussmesseinheit 150, 255 dargestellt. Dabei wird vor und hinter einer Blende, die den jeweiligen Gaszweig 110, 120 verengt, ein Druck gemessen und dadurch auf bekannte Weise auf den Gasfluss innerhalb des jeweiligen Gaszweigs 110, 120 geschlossen. Die genaue Funktionsweise eines Differenzdrucksensors ist bekannt und wird daher im Folgenden nicht näher erläutert.

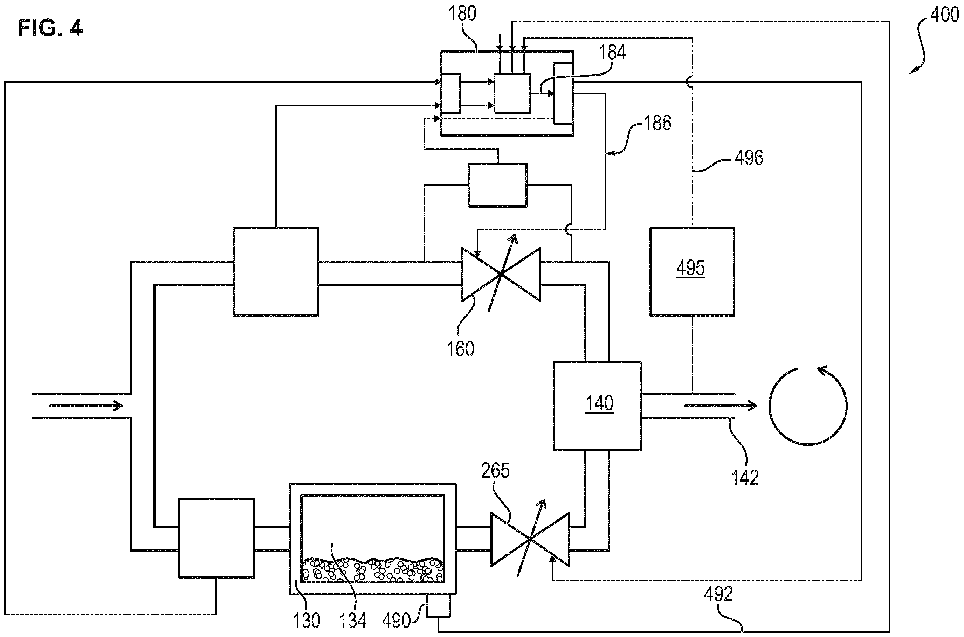

Fig. 4 zeigt eine schematische Darstellung eines vierten Ausführungsbeispiels der erfindungsgemäßen Anästhesiemitteldosiervorrichtung 400.

Die Anästhesiemitteldosiervorrichtung 400 unterscheidet sich von der in Fig. 3 dargestellten Anästhesiemitteldosiervorrichtung 300 dadurch, dass eine Temperaturmesseinheit 490 an dem Anästhesiemittelverdunster 130 vorgesehen ist, die dazu ausgebildet ist, die aktuell vorliegende Temperatur innerhalb der Verdunsterkammer 134 zu bestimmen und ein Temperatursignal 492 an die Steuereinheit 180 auszugeben, das diese aktuell vorliegende Temperatur indiziert. Die Steuereinheit 180 ist in diesem Ausführungsbeispiel weiterhin dazu ausgebildet, den Sollkammerdruck 184 auch basierend auf der aktuell vorliegenden Temperatur innerhalb der Verdunsterkammer 134 zu bestimmen.

Die Verwendung der Temperaturmesseinheit 490 gemäß diesem Ausführungsbeispiel ist eine besonders bevorzugte Realisierung der erfindungsgemäßen Anästhesiemitteldosiervorrichtung, da die thermodynamischen Bedingungen innerhalb der Verdunsterkammer 134 besonders genau bei der Bestimmung des Sollkammerdrucks 184 von der Steuereinheit 180 berücksichtigt werden können. Während in alternativen Ausführungsbeispielen eine konstante Temperatur innerhalb der Verdunsterkammer angenommen wird und/oder eine konstante Temperatur innerhalb der Verdunsterkammer durch externe Bestandteile des Anästhesiemittelgerätes sichergestellt wird, kann in der Anästhesiemitteldosiervorrichtung 400 auch die Temperatur innerhalb der Verdunsterkammer 134 veränderlich sein. Eine Veränderung der Temperatur innerhalb der Verdunsterkammer 134 sorgt lediglich für eine veränderte Berechnungsgrundlage innerhalb der Steuereinheit 180 für das Berechnen des Sollkammerdrucks 184. Die konkrete Berechnung geeigneter Druckverhältnisse innerhalb der Verdunsterkammer 134 basiert auf bekannten thermodynamischen Gesetzmäßigkeiten und ist dem Fachmann daher bekannt.

Weiterhin unterscheidet sich die Anästhesiemitteldosiervorrichtung 400 von den anderen dargestellten Anästhesiemitteldosiervorrichtungen dadurch, dass zwischen der Mischereinheit 140 und dem Auslass 142 eine Konzentrationsmesseinheit 495 angeordnet ist, die ausgebildet ist, eine aktuelle Konzentration des Anästhesiemittels im Bereich des Auslasses 142 zu bestimmen und ein entsprechendes Konzentrationssignal 496 auszugeben. Die Steuereinheit 180 ist hierbei weiterhin ausgebildet, das Konzentrationssignal 496 zu empfangen und die Sollventilöffnung 186 abhängig von der durch das Konzentrationssignal 496 indizierten aktuellen Konzentration des Anästhesiemittels im Bereich des Auslasses 142 zu bestimmen. Das Vorsehen der Konzentrationsmesseinheit 495 ermöglicht eine Kontrolle, ob die Konzentration des Anästhesiemittels am Auslass 142 tatsächlich der vorbestimmten Sollkonzentration entsprechend dem Sollkonzentrationssignal 182 entspricht. In dem dargestellten Ausführungsbeispiel ist die Steuereinheit 180 weiterhin dazu ausgebildet, die Sollventilöffnung 186 abhängig von einer Differenz zwischen der aktuell gemäß Konzentrationsmesseinheit 495 vorliegenden Konzentration und der vorbestimmten Sollkonzentration zu bestimmen.

Erfindungsgemäß ist die vorgeschlagene Anästhesiemitteldosiervorrichtung zum besonders einfachen und zuverlässigen Einstellen der Anästhesiemittelkonzentration eines einem Anästhesieatemkreis bereitzustellenden Gasgemisches ausgebildet. In diesem Ausführungsbeispiel ermöglicht die Konzentrationsmesseinheit 495 zusätzlich ein Regeln und/oder Überwachen der Anästhesiemittelkonzentration, da der Einfluss einer Veränderung der Ventilöffnung des Proportionalventils 160 und des weiteren Ventils 265 direkt zu einer Veränderung der Anästhesiemittelkonzentration am Auslass 142 führt, die dort durch die Konzentrationsmesseinheit 495 gemessen wird. So können Fehler innerhalb der Anästhesiemitteldosiervorrichtung 400, wie beispielsweise eine Leckage, besonders schnell erkannt und/oder eine fehlerhafte Steuerung durch die Steuereinheit 180 besonders schnell korrigiert werden.

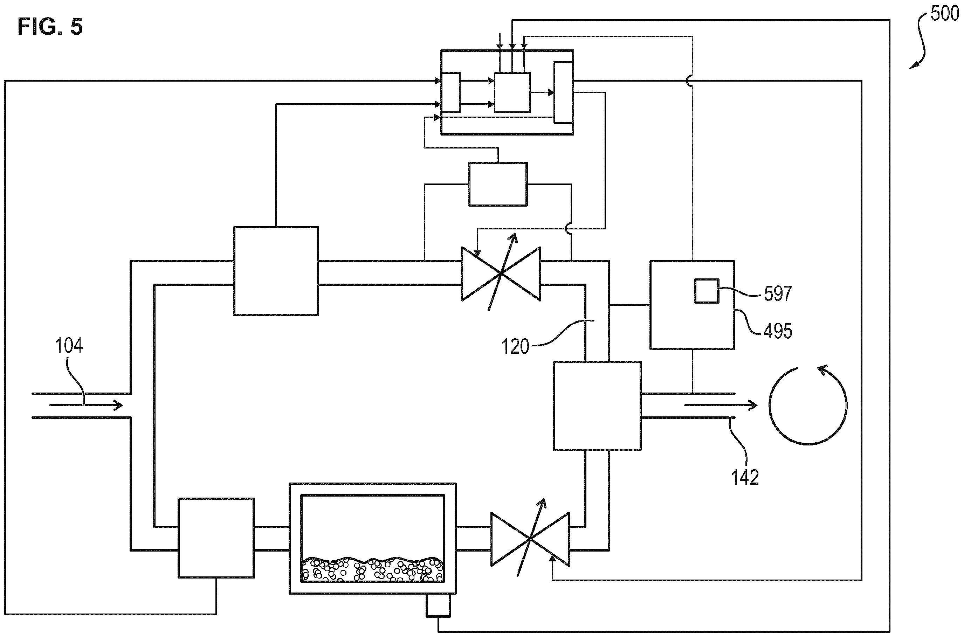

Fig. 5 zeigt eine schematische Darstellung eines fünften Ausführungsbeispiels der erfindungsgemäßen Anästhesiemitteldosiervorrichtung 500.

Die Anästhesiemitteldosiervorrichtung 500 unterscheidet sich von der in Fig. 4 dargestellten Anästhesiemitteldosiervorrichtung 400 dadurch, dass die Konzentrationsmesseinheit 495 mindestens ein Konzentrationsmessmodul 597 aufweist, das ausgebildet ist, die Konzentration des Anästhesiemittels in dem Gasgemisch 104 im Bereich des Auslasses 142 basierend auf einer Wärmeleitfähigkeitsmessung und/oder einer elektrochemischen Messung zu bestimmen.

Ein für die elektrochemische Messung der Konzentration geeigneter Sensor ist typischerweise ein Sensor, der die Konzentration des betreffenden Gases innerhalb eines Gasgemisches spezifisch bestimmen kann.

Ein für die Wärmeleitfähigkeitsmessung notwendiger Wärmeleitfähigkeitssensor ist typischerweise ein besonders einfach strukturierter Sensor, der nicht direkt eine Konzentration eines Gases innerhalb eines Gasgemisches bestimmen kann, da er lediglich einen Temperaturwert ausgibt, der eine Wärmeleitfähigkeit des vorliegenden Gases indiziert. In dem dargestellten Ausführungsbeispiel wird die Bestimmung der Konzentration über die Wärmeleitfähigkeitsmessung dadurch ermöglicht, dass die Konzentrationsmesseinheit 495 sowohl mit dem Auslass 142, als auch mit dem zweiten Gaszweig 120 pneumatisch verbunden ist. Dadurch ist das Konzentrationsmessmodul 597 derart innerhalb der Anästhesiemitteldosiervorrichtung 500 angeordnet, dass es eine Wärmeleitfähigkeitsmessung im Bereich des Auslasses 142 und in dem zweiten Gaszweig 120 gleichzeitig oder in aufeinanderfolgenden Zeitintervallen durchführen kann. Durch den Vergleich zwischen zwei Messwerten, wie beispielsweise Wärmeleitfähigkeiten oder Temperaturen, kann durch eine entsprechende Kalibrierung des Konzentrationsmessmoduls 597 die Anästhesiemittelkonzentration im Bereich des Auslasses 142 bestimmt werden. Im zweiten Gaszweig 120 liegt erfindungsgemäß kein Anästhesiemittel vor, so dass die Differenz zwischen den aufgenommenen Messwerten innerhalb des zweiten Gaszweigs 120 und des mit Anästhesiemittel beladenen Gasgemisches im Bereich des Auslasses 142 diese Anästhesiemittelkonzentration indiziert.

In einem nicht dargestellten Ausführungsbeispiel ist die Steuereinheit weiterhin ausgebildet, ein Frischgassignal zu empfangen, das einen vorbestimmten an dem Auslass bereitzustellenden Frischgasfluss des Gasgemisches indiziert. In diesem nicht dargestellten Ausführungsbeispiel ist die Steuereinheit weiterhin ausgebildet, die Sollventilöffnung abhängig von dem vorbestimmten Frischgasfluss zu bestimmen. Typischerweise wird der vorbestimmte Frischgasfluss durch eine entsprechende Nutzereingabe über eine Benutzerschnittstelle eingegeben und das entsprechende Frischgassignal durch diese Benutzerschnittstelle bereitgestellt.

In einem weiteren nicht dargestellten Ausführungsbeispiel weist die Anästhesiemitteldosiereinheit eine Benutzerschnittstelle auf, wobei die Benutzerschnittstelle ausgebildet ist, eine die Sollkonzentration betreffende Nutzereingabe zu empfangen und das entsprechende Sollkonzentrationssignal an die Steuereinheit auszugeben.

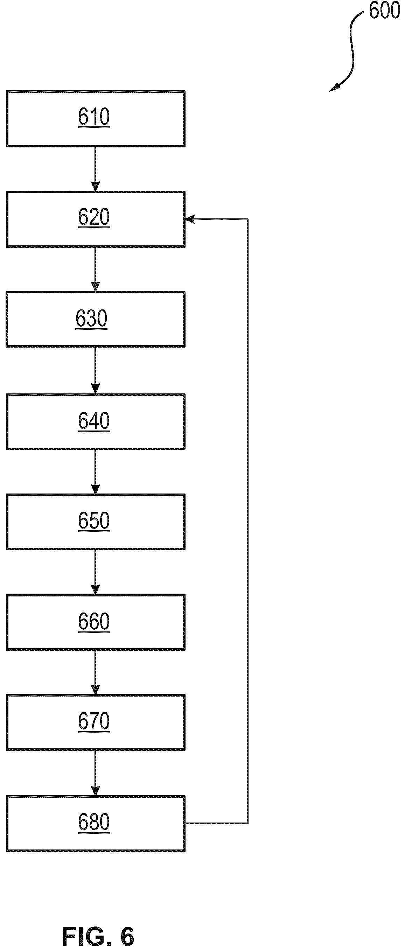

Fig. 6 zeigt ein Ablaufdiagramm eines Ausführungsbeispiels eines Verfahrens 600 gemäß dem weiteren Aspekt der Erfindung.

Das erfindungsgemäße Verfahren 600 zum Einstellen einer Anästhesiemittelkonzentration in einem einem Anästhesieatemkreis bereitzustellenden Gasgemisch weist die im Folgenden beschriebenen Schritte auf.

Ein erster Schritt 610 umfasst ein Bereitstellen eines ersten und zweiten Gaszweigs, wobei der erste Gaszweig mit einer Atemgaszufuhr verbindbar ist und ausgebildet ist, ein Atemgas durch einen Anästhesiemittelverdunster zu einer Mischereinheit zu führen, und wobei der zweite Gaszweig mit der Atemgaszufuhr verbindbar ist und ausgebildet ist, das Atemgas direkt zu der Mischereinheit zu führen, und wobei die Mischereinheit, angeordnet und ausgebildet ist, das mit einem Anästhesiemittel angereicherte Atemgas aus dem ersten Gaszweig mit dem Atemgas aus dem zweiten Gaszweig zu mischen und das dadurch entstandene Gasgemisch einem Auslass der Anästhesiemitteldosiervorrichtung bereitzustellen.

Ein darauffolgender Schritt 620 umfasst ein Empfangen eines Ventilsteuersignals und ein Einstellen einer Ventilöffnung eines Proportionalventils entsprechend dem Ventilsteuersignal.

Ein nächster Schritt 630 umfasst ein Messen eines ersten Gasflusses und ein Ausgeben eines entsprechenden ersten Gasflusssignals.

Ein weiterer Schritt 640 umfasst ein Messen eines Gasdrucks innerhalb des zweiten Gaszweigs relativ zur Flussrichtung vor dem Proportionalventil und ein Ausgeben eines entsprechenden Ventildrucksignals.

Ein darauffolgender Schritt 650 umfasst ein Empfangen des ersten Gasflusssignals und eines zweiten Gasflusssignals und ein Bestimmen eines ersten Gaszweigflusses in dem ersten Gaszweig und eines zweiten Gaszweigflusses in dem zweiten Gaszweig abhängig von dem ersten und zweiten Gasflusssignal.

Ein weiterer Schritt 660 umfasst ein Empfangen eines Sollkonzentrationssignals, das eine vorbestimmte Sollkonzentration des Anästhesiemittels am Auslass indiziert, und ein Bestimmen eines Sollkammerdrucks einer Verdunsterkammer des Anästhesiemittelverdunsters basierend auf der vorbestimmten Sollkonzentration und dem ersten und zweiten Gaszweigfluss.

Ein nächster Schritt 670 umfasst ein Bestimmen einer Sollventilöffnung des Proportionalventils abhängig von dem Sollkammerdruck und dem Ventildrucksignal, wobei die Sollventilöffnung derart bestimmt wird, dass durch die Sollventilöffnung des Proportionalventils in dem zweiten Gaszweig der Sollkammerdruck innerhalb des Anästhesiemittelverdunsters in dem ersten Gaszweig erreicht wird.

Ein darauffolgender Schritt 680 umfasst ein Ausgeben des die Sollventilöffnung indizierenden Ventilsteuersignals an das Proportionalventil.

Der Schritt 610 wird vorzugsweise bei der Herstellung der erfindungsgemäßen Anästhesiemitteldosiervorrichtung ausgeführt und daraufhin nicht wiederholt. Die Schritte 630 und 640 können in unterschiedlicher Reihenfolge ausgeführt werden und sind grundsätzlich unabhängig voneinander. Die Schritte 650, 660, 670 und 680 werden vorzugsweise in dieser Reihenfolge ausgeführt. Nach dem Schritt 680 wird das Verfahren bei dem Schritt 620 erneut begonnen mit dem jeweils neu ausgegebenen Ventilsteuersignal. Vorzugsweise werden die Verfahrensschritte 620-680 innerhalb eines Zeitintervalls ausgeführt, das kürzer als 1 Minute, insbesondere kürzer als 30 Sekunden, vorzugsweise kürzer als 10 Sekunden ist.

In einer bevorzugten Variante dieses Ausführungsbeispiels weist der Schritt 630 des vorgeschlagenen Verfahrens 600 weiterhin ein Messen eines zweiten Gasflusses und ein Ausgeben des entsprechenden zweiten Gasflusssignals auf. In dieser Variante wird vorteilhaft das zweite Gasflusssignal dynamisch im Rahmen des erfindungsgemäßen Verfahrens 600 und nicht beispielsweise durch externe Vorrichtungen oder durch eine besonders konstante Voreinstellung bereitgestellt.

In einem weiteren bevorzugten Ausführungsbeispiel umfasst das Verfahren weiterhin den Schritt eines Messens einer Temperatur innerhalb der Verdunsterkammer des Anästhesiemittelverdunsters und eines Ausgebens eines entsprechenden Temperatursignals. In diesem bevorzugten Ausführungsbeispiel wird der Schritt 660 dadurch ergänzt, dass das Bestimmen des Sollkammerdrucks weiterhin auf der gemessenen aktuellen Temperatur innerhalb der Verdunsterkammer basiert.

### Bezugszeichenliste

- 100, 200, 300, 400, 500: Anästhesiemitteldosiervorrichtung
- 104: Atemgas
- 105: Anästhesieatemkreis
- 108: Gasgemisch
- 110: erster Gaszweig
- 112: Atemgaszufuhr
- 120: zweiter Gaszweig
- 130: Anästhesiem ittelverdunster
- 132: Anästhesiem ittel
- 134: Verdunsterkammer
- 140: Mischereinheit
- 142: Auslass
- 150: erste Gasflussmesseinheit
- 152: erstes Gasflusssignal
- 153: zweiter Gaszweigfluss
- 158: zweites Gasflusssignal
- 159: erster Gaszweigfluss
- 160: Proportionalventil
- 162: Ventilsteuersignal
- 170, \370: Druckmesseinheit
- 172: Ventildrucksignal
- 180: Steuereinheit
- 182: Sollkonzentrationssignal
- 184: Sollkammerdruck
- 186: Sollventilöffnung
- 255: zweite Gasflussmesseinheit
- 265: weiteres Ventil
- 267: weiteres Ventilsteuersignal
- 490: Temperatursensor
- 492: Temperatursignal
- 495: Konzentrationsmesseinheit
- 496: Konzentrationssignal
- 597: Konzentrationsmessmodul
- 600: Verfahren
- 610, 620, 630, 640, 650,: Verfahrensschritte 660, 670, 680

## Patentansprüche

1. Anästhesiemitteldosiervorrichtung (100) zum Einstellen einer Anästhesiemittelkonzentration in einem einem Anästhesieatemkreis (105) bereitzustellenden Gasgemisch (108), mit
- einem ersten Gaszweig (110), der mit einer Atemgaszufuhr (112) verbindbar ist und der ausgebildet ist, ein Atemgas (104) durch einen Anästhesiemittelverdunster (130) zu einer Mischereinheit (140) zu führen,
- einem zweiten Gaszweig (120), der mit der Atemgaszufuhr (112) verbindbar ist und der ausgebildet ist, das Atemgas (104) direkt zu der Mischereinheit (140) zu führen,
- dem Anästhesiemittelverdunster (130), der angeordnet und ausgebildet ist, das Atemgas (104) in dem ersten Gaszweig (110) mit einem Anästhesiemittel (132) anzureichern,
- der Mischereinheit (140), die angeordnet und ausgebildet ist, das mit dem Anästhesiemittel (132) angereicherte Atemgas (104) aus dem ersten Gaszweig (110) mit dem Atemgas (104) aus dem zweiten Gaszweig (120) zu mischen und das dadurch entstandene Gasgemisch (108) einem Auslass (142) der Anästhesiemitteldosiervorrichtung (100) bereitzustellen,
- einer ersten Gasflussmesseinheit (150), die ausgebildet ist, einen ersten Gasfluss zu messen und ein entsprechendes erstes Gasflusssignal (152) auszugeben,
- einem Proportionalventil (160), das in dem zweiten Gaszweig (120) angeordnet ist und dazu ausgebildet ist, ein Ventilsteuersignal (162) zu empfangen und entsprechend dem Ventilsteuersignal (162) eine Ventilöffnung einzustellen,
- einer Druckmesseinheit (170), die angeordnet und ausgebildet ist, einen Gasdruck innerhalb des zweiten Gaszweigs (120) zu messen und ein entsprechendes Ventildrucksignal (172) auszugeben, und
- einer Steuereinheit (180), die ausgebildet ist, das Ventildrucksignal (172), das erste Gasflusssignal (152) und ein zweites Gasflusssignal (158) zu empfangen und abhängig von dem ersten und zweiten Gasflusssignal (152, 158) einen ersten Gaszweigfluss (159) in dem ersten Gaszweig (110) und einen zweiten Gaszweigfluss (153) in dem zweiten Gaszweig (120) zu bestimmen, wobei die Steuereinheit (180) weiterhin ausgebildet ist, ein Sollkonzentrationssignal (182) zu empfangen, das eine vorbestimmte Sollkonzentration des Anästhesiemittels (132) am Auslass (142) indiziert, und basierend auf der vorbestimmten Sollkonzentration und dem ersten und zweiten Gaszweigfluss (159, 153) einen Sollkammerdruck (184) einer Verdunsterkammer (134) des Anästhesiemittelverdunsters (130) zu bestimmen, wobei die Steuereinheit (180) weiterhin ausgebildet ist, abhängig von dem Sollkammerdruck (184) und dem Ventildrucksignal (172) eine Sollventilöffnung (186) des Proportionalventils (160) zu bestimmen, wobei die Sollventilöffnung (186) derart bestimmt ist, dass durch die Sollventilöffnung (186) des Proportionalventils (160) der Sollkammerdruck (184) innerhalb des Anästhesiemittelverdunsters (130) in dem ersten Gaszweig (110) erreicht wird, und wobei die Steuereinheit (180) weiterhin ausgebildet ist, das die Sollventilöffnung (186) indizierende Ventilsteuersignal (162) an das Proportionalventil (160) auszugeben.

2. Anästhesiemitteldosiervorrichtung (200) gemäß Anspruch 1, weiterhin aufweisend eine zweite Gasflussmesseinheit (255), die ausgebildet ist, einen zweiten Gasfluss zu bestimmen und basierend auf dem zweiten Gasfluss das zweite Gasflusssignal (158) auszugeben.

3. Anästhesiemitteldosiervorrichtung (200) gemäß Anspruch 2, wobei die zweite Gasflussmesseinheit (255) in dem ersten Gaszweig (110) und die erste Gasflussmesseinheit (150) in dem zweiten Gaszweig (120) angeordnet ist.

4. Anästhesiemitteldosiervorrichtung (200) gemäß mindestens einem der vorhergehenden Ansprüche, wobei die erste Gasflussmesseinheit (150) und/oder die zweite Gasflussmesseinheit (255) einen Differenzdrucksensor umfasst.

5. Anästhesiemitteldosiervorrichtung (100) gemäß mindestens einem der vorhergehenden Ansprüche, wobei die Druckmesseinheit (170) relativ zur Flussrichtung zumindest teilweise vor dem Proportionalventil (160) angeordnet ist.

6. Anästhesiemitteldosiervorrichtung (300) gemäß Anspruch 5, wobei die Druckmesseinheit (370) weiterhin ausgebildet ist, relativ zur Flussrichtung hinter dem Proportionalventil (160) den Gasdruck innerhalb des zweiten Gaszweigs (120) zu messen.

7. Anästhesiemitteldosiervorrichtung (200) gemäß mindestens einem der vorhergehenden Ansprüche, weiterhin aufweisend ein weiteres Ventil (265), das in dem ersten Gaszweig (110) relativ zur Flussrichtung hinter dem Anästhesiemittelverdunster (130) angeordnet ist und das ausgebildet ist, ein weiteres Ventilsteuersignal (267) zu empfangen und entsprechend des weiteren Ventilsteuersignals (267) eine weitere Ventilöffnung des weiteren Ventils (265) einzustellen.

8. Anästhesiemitteldosiervorrichtung (200) gemäß Anspruch 7, wobei das weitere Ventil (265) ein weiteres Proportionalventil ist.

9. Anästhesiemitteldosiervorrichtung (100) gemäß mindestens einem der vorhergehenden Ansprüche, wobei das Ventilsteuersignal (162) derart bereitgestellt ist, dass ein vorbestimmter Mindestdruck innerhalb der Verdunsterkammer (134) des Anästhesiemittelverdunsters (130) nicht unterschritten wird.

10. Anästhesiemitteldosiervorrichtung (400) gemäß mindestens einem der vorhergehenden Ansprüche, weiterhin aufweisend eine zwischen Mischereinheit (140) und Auslass (142) angeordnete Konzentrationsmesseinheit (495), die ausgebildet ist, eine aktuelle Konzentration des Anästhesiemittels (132) zu bestimmen und ein entsprechendes Konzentrationssignal (496) auszugeben, wobei die Steuereinheit (180) weiterhin ausgebildet ist, das Konzentrationssignal (496) zu empfangen und die Sollventilöffnung (186) abhängig von der durch das Konzentrationssignal (496) indizierten aktuellen Konzentration zu bestimmen.

11. Anästhesiemitteldosiervorrichtung (500) gemäß Anspruch 10, wobei die Konzentrationsmesseinheit (495) mindestens ein Konzentrationsmessmodul (597) aufweist, das ausgebildet ist, die Konzentration des Anästhesiemittels (132) in dem Gasgemisch (108) basierend auf einer Wärmeleitfähigkeitsmessung und/oder auf einer elektro-chemischen Messung zu bestimmen.

12. Anästhesiemitteldosiervorrichtung (400, 500) gemäß Anspruch 10 oder 11, wobei die Steuereinheit (180) ausgebildet ist, die Sollventilöffnung (186) abhängig von einer Differenz zwischen aktueller Konzentration und vorbestimmter Sollkonzentration zu bestimmen.

13. Anästhesiemitteldosiervorrichtung (400) gemäß mindestens einem der vorhergehenden Ansprüche, wobei der Anästhesiemittelverdunster (130) weiterhin eine Temperaturmesseinheit (490) aufweist, die ausgebildet ist, eine Temperatur innerhalb der Verdunsterkammer (134) des Anästhesiemittelverdunsters (130) zu bestimmen und ein Temperatursignal (492) auszugeben, dass die innerhalb der Verdunsterkammer vorliegende Temperatur indiziert, und wobei die Steuereinheit (180) weiterhin ausgebildet, das Temperatursignal (492) zu empfangen und den Sollkammerdruck (184) abhängig von dem Temperatursignal (492) zu bestimmen.

14. Verfahren (600) zum Einstellen einer Anästhesiemittelkonzentration in einem einem Anästhesieatemkreis (105) bereitzustellenden Gasgemisch (108), aufweisend die Schritte:
- Bereitstellen eines ersten und zweiten Gaszweigs (110, 120), wobei der erste Gaszweig (110) mit einer Atemgaszufuhr (112) verbindbar ist und ausgebildet ist, ein Atemgas (104) durch einen Anästhesiemittelverdunster (130) zu einer Mischereinheit (140) zu führen, und wobei der zweite Gaszweig (120) mit der Atemgaszufuhr (112) verbindbar ist und ausgebildet ist, das Atemgas (104) direkt zu der Mischereinheit (140) zu führen, und wobei die Mischereinheit (140), angeordnet und ausgebildet ist, das mit einem Anästhesiemittel (132) angereicherte Atemgas (104) aus dem ersten Gaszweig (110) mit dem Atemgas (104) aus dem zweiten Gaszweig (120) zu mischen und das dadurch entstandene Gasgemisch (108) einem Auslass (142) der Anästhesiemitteldosiervorrichtung (100) bereitzustellen;
- Empfangen eines Ventilsteuersignals (162) und Einstellen einer Ventilöffnung eines Proportionalventils (160) entsprechend dem Ventilsteuersignal (162);
- Messen eines ersten Gasflusses und Ausgeben eines entsprechenden ersten Gasflusssignals (152);
- Messen eines Gasdrucks innerhalb des zweiten Gaszweigs (120) und Ausgeben eines entsprechenden Ventildrucksignals (172);
- Empfangen des ersten Gasflusssignals (152) und eines zweiten Gasflusssignals (158) und Bestimmen eines ersten Gaszweigflusses (159) in dem ersten Gaszweig (110) und eines zweiten Gaszweigflusses (153) in dem zweiten Gaszweig (120) abhängig von dem ersten und zweiten Gasflusssignal (152, 158);
- Empfangen eines Sollkonzentrationssignals (182), das eine vorbestimmte Sollkonzentration des Anästhesiemittels (132) am Auslass (142) indiziert, und Bestimmen eines Sollkammerdrucks (184) einer Verdunsterkammer (134) des Anästhesiemittelverdunsters (130) basierend auf der vorbestimmten Sollkonzentration und dem ersten und zweiten Gaszweigfluss (159, 153);
- Bestimmen einer Sollventilöffnung (186) des Proportionalventils (160) abhängig von dem Sollkammerdruck (184) und dem Ventildrucksignal (172), wobei die Sollventilöffnung (186) derart bestimmt wird, dass durch die Sollventilöffnung (186) des Proportionalventils (160) in dem zweiten Gaszweig (120) der Sollkammerdruck (184) innerhalb des Anästhesiemittelverdunsters (130) in dem ersten Gaszweig (110) erreicht wird;
- Ausgeben des die Sollventilöffnung (186) indizierenden Ventilsteuersignals (162) an das Proportionalventil (160).

## Claims

1. Anaesthetic agent metering device (100) for adjusting an anaesthetic agent concentration in a gas mixture (108) to be provided to an anaesthetic breathing circuit (105), having
- a first gas branch (110) which is connectable to a breathing gas supply (112) and is designed to conduct a breathing gas (104) through an anaesthetic agent evaporator (130) to a mixer unit (140),
- a second gas branch (120) which is connectable to the breathing gas supply (112) and is designed to conduct the breathing gas (104) directly to the mixer unit (140),
- the anaesthetic agent evaporator (130) which is arranged and designed to enrich the breathing gas (104) in the first gas branch (110) with an anaesthetic agent (132),
- the mixer unit (140) which is arranged and designed to mix the breathing gas (104), which is enriched with the anaesthetic agent (132), from the first gas branch (110) with the breathing gas (104) from the second gas branch (120) and to provide the resulting gas mixture (108) to an outlet (142) of the anaesthetic agent metering device (100),
- a first gas-flow-measuring unit (150) which is designed to measure a first gas flow and to output a corresponding first gas flow signal (152),
- a proportional valve (160) which is arranged in the second gas branch (120) and is designed to receive a valve control signal (162) and to adjust a valve opening in accordance with the valve control signal (162),
- a pressure-measuring unit (170) which is arranged and designed to measure a gas pressure within the second gas branch (120) and to output a corresponding valve pressure signal (172), and
- a control unit (180) which is designed to receive the valve pressure signal (172), the first gas flow signal (152) and a second gas flow signal (158) and, depending on the first and second gas flow signal (152, 158), to determine a first gas branch flow (159) in the first gas branch (110) and a second gas branch flow (153) in the second gas branch (120), wherein the control unit (180) is furthermore designed to receive a desired concentration signal (182) which indicates a predetermined desired concentration of the anaesthetic agent (132) at the outlet (142), and, on the basis of the predetermined desired concentration and the first and second gas branch flow (159, 153), to determine a desired chamber pressure (184) of an evaporator chamber (134) of the anaesthetic agent evaporator (130), wherein the control unit (180) is furthermore designed to determine, depending on the desired chamber pressure (184) and the valve pressure signal (172), a desired valve opening (186) of the proportional valve (160), wherein the desired valve opening (186) is determined in such a manner that, through the desired valve opening (186) of the proportional valve (160), the desired chamber pressure (184) within the anaesthetic agent evaporator (130) in the first gas branch (110) is reached, and wherein the control unit (180) is furthermore designed to output the valve control signal (162) indicating the desired valve opening (186) to the proportional valve (160).

2. Anaesthetic agent metering device (200) according to Claim 1, furthermore having a second gas-flow- measuring unit (255) which is designed to determine a second gas flow and, on the basis of the second gas flow, to output the second gas flow signal (158).

3. Anaesthetic agent metering device (200) according to Claim 2, wherein the second gas-flow-measuring unit (255) is arranged in the first gas branch (110) and the first gas-flow-measuring unit (150) is arranged in the second gas branch (120).

4. Anaesthetic agent metering device (200) according to at least one of the preceding claims, wherein the first gas-flow-measuring unit (150) and/or the second gas-flow-measuring unit (255) comprises a differential pressure sensor.

5. Anaesthetic agent metering device (100) according to at least one of the preceding claims, wherein the pressure-measuring unit (170) is at least partially arranged upstream of the proportional valve (160) relative to the flow direction.

6. Anaesthetic agent metering device (300) according to Claim 5, wherein the pressure-measuring unit (370) is furthermore designed to measure the gas pressure within the second gas branch (120) downstream of the proportional valve (160) relative to the flow direction.

7. Anaesthetic agent metering device (200) according to at least one of the preceding claims, furthermore having a further valve (265) which is arranged in the first gas branch (110) downstream of the anaesthetic agent evaporator (130) relative to the flow direction and which is designed to receive a further valve control signal (267) and, in accordance with the further valve control signal (267), to adjust a further valve opening of the further valve (265).

8. Anaesthetic agent metering device (200) according to Claim 7, wherein the further valve (265) is a further proportional valve.

9. Anaesthetic agent metering device (100) according to at least one of the preceding claims, wherein the valve control signal (162) is provided in such a manner that the pressure does not fall below a predetermined minimum pressure within the evaporator chamber (134) of the anaesthetic agent evaporator (130).

10. Anaesthetic agent metering device (400) according to at least one of the preceding claims, furthermore having a concentration-measuring unit (495) which is arranged between the mixer unit (140) and the outlet (142) and which is designed to determine a current concentration of the anaesthetic agent (132) and to output a corresponding concentration signal (496), wherein the control unit (180) is furthermore designed to receive the concentration signal (496) and to determine the desired valve opening (186) depending on the current concentration indicated by the concentration signal (496).

11. Anaesthetic agent metering device (500) according to Claim 10, wherein the concentration-measuring unit (495) has at least one concentration-measuring module (597) which is designed to determine the concentration of the anaesthetic agent (132) in the gas mixture (108) on the basis of a heat conductivity measurement and/or on the basis of an electrochemical measurement.

12. Anaesthetic agent metering device (400, 500) according to Claim 10 or 11, wherein the control unit (180) is designed to determine the desired valve opening (186) depending on a difference between the current concentration and the predetermined desired concentration.

13. Anaesthetic agent metering device (400) according to at least one of the preceding claims, wherein the anaesthetic agent evaporator (130) furthermore has a temperature-measuring unit (490) which is designed to determine a temperature within the evaporator chamber (134) of the anaesthetic agent evaporator (130) and to output a temperature signal (492) which indicates the temperature which is present within the evaporator chamber, and wherein the control unit (180) is furthermore designed to receive the temperature signal (492) and to determine the desired chamber pressure (184) depending on the temperature signal (492).

14. Method (600) for adjusting an anaesthetic agent concentration in a gas mixture (108) to be provided to an anaesthetic breathing circuit (105), comprising the following steps:
- providing a first and a second gas branch (110, 120), wherein the first gas branch (110) is connectable to a breathing gas supply (112) and is designed to conduct a breathing gas (104) through an anaesthetic agent evaporator (130) to a mixer unit (140), and wherein the second gas branch (120) is connectable to the breathing gas supply (112) and is designed to conduct the breathing gas (104) directly to the mixer unit (140), and wherein the mixer unit (140) is arranged and designed to mix the breathing gas (104), which is enriched with an anaesthetic agent (132), from the first gas branch (110) with the breathing gas (104) from the second gas branch (120) and to provide the resulting gas mixture (108) to an outlet (142) of the anaesthetic agent metering device (100);
- receiving a valve control signal (162) and adjusting a valve opening of a proportional valve (160) in accordance with the valve control signal (162);
- measuring a first gas flow and outputting a corresponding first gas flow signal (152);
- measuring a gas pressure within the second gas branch (120) and outputting a corresponding valve pressure signal (172);
- receiving the first gas flow signal (152) and a second gas flow signal (158) and determining a first gas branch flow (159) in the first gas branch (110) and a second gas branch flow (153) in the second gas branch (120) depending on the first and second gas flow signal (152, 158);
- receiving a desired concentration signal (182) which indicates a predetermined desired concentration of the anaesthetic agent (132) at the outlet (142), and determining a desired chamber pressure (184) of an evaporator chamber (134) of the anaesthetic agent evaporator (130) on the basis of the predetermined desired concentration and the first and second gas branch flow (159, 153);
- determining a desired valve opening (186) of the proportional valve (160) depending on the desired chamber pressure (184) and the valve pressure signal (172), wherein the desired valve opening (186) is determined in such a manner that, through the desired valve opening (186) of the proportional valve (160) in the second gas branch (120), the desired chamber pressure (184) within the anaesthetic agent evaporator (130) in the first gas branch (110) is reached;
- outputting the valve control signal (162) indicating the desired valve opening (186) to the proportional valve (160).

## Revendications

1. Dispositif (100) de dosage d'agent anesthésique, dévolu au réglage d'une concentration en agent anesthésique dans un mélange gazeux (108) devant être fourni à un circuit respiratoire anesthésique (105), comprenant
- un premier embranchement (110) de circulation gazeuse, pouvant être raccordé à une amenée (112) de gaz respiratoire et conçu pour guider un gaz respiratoire (104) vers une unité de mélange (140), par l'intermédiaire d'un évaporateur (130) d'agent anesthésique,
- un second embranchement (120) de circulation gazeuse, pouvant être raccordé à ladite amenée (112) de gaz respiratoire et conçu pour guider directement ledit gaz respiratoire (104) vers ladite unité de mélange (140),
- ledit évaporateur (130) d'agent anesthésique, disposé et conçu de façon à enrichir ledit gaz respiratoire (104) par un agent anesthésique (132), dans ledit premier embranchement (110) de circulation gazeuse,
- ladite unité de mélange (140), disposée et conçue de façon à mélanger le gaz respiratoire (104), enrichi par l'agent anesthésique (132) et provenant du premier embranchement (110) de circulation gazeuse, au gaz respiratoire (104) émanant du second embranchement (120) de circulation gazeuse, et de façon à fournir le mélange gazeux (108), ainsi produit, à une sortie (142) dudit dispositif (100) de dosage d'agent anesthésique,
- une première unité (150) de mesure de flux gazeux, conçue pour mesurer un premier flux gazeux et pour délivrer un premier signal correspondant (152) de flux gazeux,
- une valve proportionnelle (160), intégrée dans le second embranchement (120) de circulation gazeuse et conçue pour recevoir un signal de commande (162) et pour régler une ouverture de ladite valve en concordance avec ledit signal (162) de commande de ladite valve,
- une unité (170) de mesure de pressions, disposée et conçue pour mesurer une pression gazeuse, à l'intérieur dudit second embranchement (120) de circulation gazeuse, et pour délivrer un signal correspondant (172) de pression de distribution, et
- une unité de commande (180) conçue pour recevoir ledit signal (172) de pression de distribution, ledit premier signal (152) de flux gazeux et un second signal (158) de flux gazeux et pour déterminer, en fonction des premier et second signaux (152, 158) de flux gazeux, un premier flux (159) parcourant le premier embranchement (110) de circulation gazeuse et un second flux (153) parcourant le second embranchement (120) de circulation gazeuse, l'unité de commande (180) étant conçue, en outre, de façon à recevoir un signal (182) de concentration de consigne indicatif d'une concentration de consigne prédéterminée de l'agent anesthésique (132) au niveau de la sortie (142), et de façon à déterminer, sur la base de ladite concentration de consigne prédéterminée et des premier et second flux (159, 153) parcourant lesdits embranchements de circulation gazeuse, une pression de consigne (184) d'une chambre d'évaporation (134) de l'évaporateur (130) d'agent anesthésique, sachant que ladite unité de commande (180) est par ailleurs conçue pour déterminer une ouverture de consigne (186) de la valve proportionnelle (160), en fonction de ladite pression de consigne (184) régnant dans la chambre et dudit signal (172) de pression de distribution, laquelle ouverture de consigne (186) est déterminée de telle sorte que, par l'intermédiaire de ladite ouverture de consigne (186) de ladite valve proportionnelle (160), ladite pression de consigne (184) régnant dans la chambre soit atteinte à l'intérieur dudit évaporateur (130) d'agent anesthésique, dans le premier embranchement (110) de circulation gazeuse, et sachant que ladite unité de commande (180) est de surcroît conçue pour délivrer, à ladite valve proportionnelle (160), le signal (162) de commande de ladite valve indicatif de ladite ouverture de consigne (186) de ladite valve.

2. Dispositif (200) de dosage d'agent anesthésique, selon la revendication 1, comportant par ailleurs une seconde unité (255) de mesure de flux gazeux, conçue pour déterminer un second flux gazeux et pour délivrer le second signal (158) de flux gazeux, sur la base dudit second flux gazeux.

3. Dispositif (200) de dosage d'agent anesthésique, selon la revendication 2, la seconde unité (255) de mesure de flux gazeux étant intégrée dans le premier embranchement (110) de circulation gazeuse, et la première unité (150) de mesure de flux gazeux étant intégrée dans le second embranchement (120) de circulation gazeuse.

4. Dispositif (200) de dosage d'agent anesthésique, selon au moins l'une des revendications précédentes, la première unité (150) de mesure de flux gazeux et/ou la seconde unité (255) de mesure de flux gazeux incluant un capteur de pressions différentielles.

5. Dispositif (100) de dosage d'agent anesthésique, selon au moins l'une des revendications précédentes, l'unité (170) de mesure de pressions étant implantée, au moins en partie, devant la valve proportionnelle (160) par rapport à la direction de l'écoulement.

6. Dispositif (300) de dosage d'agent anesthésique, selon la revendication 5, l'unité (370) de mesure de pressions étant par ailleurs conçue pour mesurer la pression gazeuse, à l'intérieur du second embranchement (120) de circulation gazeuse, derrière la valve proportionnelle (160) par rapport à la direction de l'écoulement.

7. Dispositif (200) de dosage d'agent anesthésique, selon au moins l'une des revendications précédentes, comportant par ailleurs une valve supplémentaire (265) qui est implantée, dans le premier embranchement (110) de circulation gazeuse, derrière l'évaporateur (130) d'agent anesthésique par rapport à la direction de l'écoulement, et qui est conçue de façon à recevoir un signal de commande (267) supplémentaire et de façon à régler une ouverture de ladite valve supplémentaire (265) en concordance avec ledit signal supplémentaire (267) de commande de ladite valve.

8. Dispositif (200) de dosage d'agent anesthésique, selon la revendication 7, la valve supplémentaire (265) étant une valve proportionnelle supplémentaire.

9. Dispositif (100) de dosage d'agent anesthésique, selon au moins l'une des revendications précédentes, le signal (162) de commande de valve étant élaboré de façon telle qu'une pression minimale prédéterminée ne soit pas dépassée négativement à l'intérieur de la chambre d'évaporation (134) de l'évaporateur (130) d'agent anesthésique.

10. Dispositif (400) de dosage d'agent anesthésique, selon au moins l'une des revendications précédentes, comportant par ailleurs une unité (495) de mesure de concentrations qui est interposée entre l'unité de mélange (140) et la sortie (142), et est conçue de façon à déterminer une concentration effective de l'agent anesthésique (132) et de façon à délivrer un signal de concentration (496) correspondant, l'unité de commande (180) étant conçue, en outre, pour recevoir ledit signal de concentration (496) et pour déterminer l'ouverture de valve de consigne (186) en fonction de ladite concentration effective indiquée par ledit signal de concentration (496).

11. Dispositif (500) de dosage d'agent anesthésique, selon la revendication 10, l'unité (495) de mesure de concentrations étant munie d'au moins un module (597) mesureur de concentrations qui est conçu pour déterminer la concentration de l'agent anesthésique (132), dans le mélange gazeux (108), sur la base d'un mesurage de conductivité thermique et/ou d'un mesurage électrochimique.

12. Dispositif (400, 500) de dosage d'agent anesthésique, selon la revendication 10 ou 11, l'unité de commande (180) étant conçue pour déterminer l'ouverture de valve de consigne (186) en fonction d'une différence entre une concentration effective et une concentration de consigne prédéterminée.

13. Dispositif (400) de dosage d'agent anesthésique, selon au moins l'une des revendications précédentes, l'évaporateur (130) d'agent anesthésique étant doté, par ailleurs, d'une unité (490) de mesure de températures qui est conçue pour déterminer une température, à l'intérieur de la chambre d'évaporation (134) dudit évaporateur (130) d'agent anesthésique, et pour délivrer un signal de température (492) indicatif de la température régnant à l'intérieur de ladite chambre d'évaporation, et l'unité de commande (180) étant conçue, en outre, pour recevoir ledit signal de température (492) et pour déterminer, en fonction dudit signal de température (492), la pression de consigne (184) régnant dans ladite chambre.

14. Procédé (600) de réglage d'une concentration en agent anesthésique dans un mélange gazeux (108) devant être fourni à un circuit respiratoire anesthésique (105), incluant les étapes consistant à :
- fournir des premier et second embranchements (110, 120) de circulation gazeuse, sachant que le premier embranchement (110) de circulation gazeuse peut être raccordé à une amenée (112) de gaz respiratoire et est conçu pour guider un gaz respiratoire (104) vers une unité de mélange (140), par l'intermédiaire d'un évaporateur (130) d'agent anesthésique, sachant que le second embranchement (120) de circulation gazeuse peut être raccordé à ladite amenée (112) de gaz respiratoire et est conçu pour guider directement ledit gaz respiratoire (104) vers ladite unité de mélange (140), et sachant que ladite unité de mélange (140) est disposée et conçue de façon à mélanger le gaz respiratoire (104), enrichi par un agent anesthésique (132) et provenant du premier embranchement (110) de circulation gazeuse, au gaz respiratoire (104) émanant du second embranchement (120) de circulation gazeuse, et de façon à fournir le mélange gazeux (108), ainsi produit, à une sortie (142) dudit dispositif (100) de dosage d'agent anesthésique ;
- recevoir un signal (162) de commande de valve, et régler une ouverture d'une valve proportionnelle (160) en concordance avec ledit signal de commande (162) ;
- mesurer un premier flux gazeux et délivrer un premier signal correspondant (152) de flux gazeux ;
- mesurer une pression gazeuse à l'intérieur du second embranchement (120) de circulation gazeuse, et délivrer un signal correspondant (172) de pression de distribution ;
- recevoir ledit premier signal (152) de flux gazeux et un second signal (158) de flux gazeux et déterminer, en fonction des premier et second signaux (152, 158) de flux gazeux, un premier flux (159) parcourant le premier embranchement (110) de circulation gazeuse et un second flux (153) parcourant le second embranchement (120) de circulation gazeuse ;
- recevoir un signal (182) de concentration de consigne indicatif d'une concentration de consigne prédéterminée de l'agent anesthésique (132) au niveau de la sortie (142), et déterminer, sur la base de ladite concentration de consigne prédéterminée et des premier et second flux (159, 153) parcourant lesdits embranchements de circulation gazeuse, une pression de consigne (184) d'une chambre d'évaporation (134) de l'évaporateur (130) d'agent anesthésique ;
- déterminer une ouverture de consigne (186) de la valve proportionnelle (160), en fonction de ladite pression de consigne (184) régnant dans la chambre et dudit signal (172) de pression de distribution, laquelle ouverture de consigne (186) est déterminée de telle sorte que, par l'intermédiaire de ladite ouverture de consigne (186) de ladite valve proportionnelle (160) dans le second embranchement (120) de circulation gazeuse, ladite pression de consigne (184) régnant dans la chambre soit atteinte à l'intérieur dudit évaporateur (130) d'agent anesthésique, dans le premier embranchement (110) de circulation gazeuse ;
- délivrer, à ladite valve proportionnelle (160), le signal de commande (162) indicatif de l'ouverture de consigne (186) de ladite valve.
